(19) Europäisches Patentamt
European Patent Office
Office européen des brevets

(11) **EP 1 691 887 B1**

(12) **EUROPÄISCHE PATENTSCHRIFT**

(45) Veröffentlichungstag und Bekanntmachung des Hinweises auf die Patenterteilung:
**27.05.2009 Patentblatt 2009/22**

(21) Anmeldenummer: **04802626.4**

(22) Anmeldetag: **20.10.2004**

(51) Int Cl.:
***A61N 1/05*** *(2006.01)* ***A61N 1/06*** *(2006.01)*
***A61N 1/08*** *(2006.01)* ***A61N 1/18*** *(2006.01)*
***A61N 1/36*** *(2006.01)*

(86) Internationale Anmeldenummer:
**PCT/DE2004/002336**

(87) Internationale Veröffentlichungsnummer:
**WO 2005/053787 (16.06.2005 Gazette 2005/24)**

(54) **VORRICHTUNG ZUR DESYNCHRONISATION NEURONALER HIRNAKTIVITÄT**

DEVICE FOR DESYNCHRONISING NEURONAL CEREBRAL ACTIVITY

DISPOSITIF POUR DESYNCHRONISER L'ACTIVITE CEREBRALE NEURONALE

(84) Benannte Vertragsstaaten:
**AT BE BG CH CY CZ DE DK EE ES FI FR GB GR HU IE IT LI LU MC NL PL PT RO SE SI SK TR**

(30) Priorität: **28.11.2003 DE 10355652**

(43) Veröffentlichungstag der Anmeldung:
**23.08.2006 Patentblatt 2006/34**

(73) Patentinhaber: **Forschungszentrum Jülich GmbH
52425 Jülich (DE)**

(72) Erfinder:
- **TASS, Peter
40221 Düsseldorf (DE)**
- **POPOVYCH, Oleksandr
52355 Düren (DE)**
- **HAUPTMANN, Christian
52224 Stolberg (DE)**

(74) Vertreter: **Patentanwälte Lambsdorff & Lange
Dingolfinger Strasse 6
81673 München (DE)**

(56) Entgegenhaltungen:
**WO-A-03/077985**

- **TASS P A: "Desynchronizing double-pulse phase resetting and application to deep brain stimulation" BIOLOGICAL CYBERNETICS, SPRINGER VERLAG, BERLIN, DE, Bd. 85, Nr. 5, November 2001 (2001-11), Seiten 343-354, XP002245895 ISSN: 0340-1200**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft eine Vorrichtung zur Desynchronisation von neuronaler Hirnaktivität nach dem Oberbegriff des Anspruchs 1.

Bei Patienten mit neurologischen oder psychiatrischen Erkrankungen, z. B. Morbus Parkinson, essentiellem Tremor, Dystonie oder Zwangserkrankungen, sind Nervenzellverbände in umschriebenen Bereichen des Gehirns, z. B. des Thalamus und der Basalganglien krankhaft aktiv, z. B. übersteigert synchron. In diesem Fall bildet eine große Anzahl von Neuronen synchrone Aktionspotentiale aus ;die beteiligten Neuronen feuern übermäßig synchron. Beim Gesunden hingegen feuern die Neuronen in diesen Hirngebieten qualitativ anders, z. B. auf unkorrelierte Weise.

Beim Morbus Parkinson verändert die pathologisch synchrone Aktivität z. B. des Thalamus und der Basalganglien die neuronale Aktivität in anderen Hirngebieten, z. B. in Arealen der Großhirnrinde wie dem primär motorischen Cortex. Dabei zwingt die pathologisch synchrone Aktivität im Bereich des Thalamus und der Basalganglien beispielsweise den Großhirnarealen ihren Rhythmus auf, so dass schließlich die von diesen Arealen gesteuerten Muskeln pathologische Aktivität, z. B. ein rhythmisches Zittern (Tremor) entfalten.

Bei Patienten, welche medikamentös nicht (mehr) behandelt werden können, wird, je nach Krankheitsbild und je nach dem, ob die Erkrankung einseitig oder beidseitig auftritt, eine Tiefenelektrode einseitig oder beidseitig implantiert. Unter der Haut führt dabei ein Kabel vom Kopf zum sogenannten Generator, welcher ein Steuergerät mit einer Batterie umfasst, und beispielsweise im Bereich des Schlüsselbeins unter der Haut implantiert ist. Über die Tiefenelektroden wird eine Dauerreizung mit einer hochfrequenten periodischen Abfolge (pulse train mit einer Frequenz von > 100 Hz) von Einzelpulsen, z. B. Rechteckpulsen, durchgeführt. Ziel dieser Methode ist es, das Feuern der Neuronen in den Zielgebieten zu unterdrücken. Der Wirkmechanismus, welcher der Standard-Tiefenstimulation zugrunde liegt, ist noch nicht hinreichend geklärt. Die Ergebnisse mehrerer Studien sprechen dafür, dass die Standard-Tiefenstimulation wie eine reversible Läsionierung, d. h. wie eine reversible Ausschaltung des Gewebes, wirkt: Die Standard-Tiefenstimulation unterdrückt das Feuern der Neuronen in den Zielgebieten und/oder in damit verbundenen Hirnarealen.

Nachteilig bei dieser Stimulationsform ist, dass der Energieverbrauch des Generators sehr hoch ist, so dass der Generator inklusive Batterie häufig schon nach ca. ein bis drei Jahren operativ ausgetauscht werden muss. Noch nachteiliger ist, dass die Hochfrequenz-Dauerstimulation als unphysiologischer (unnatürlicher) Input im Bereich des Gehirns, z. B. des Thalamus bzw. der Basalganglien, im Laufe von wenigen Jahren zur Adaptation der betroffenen Nervenzellverbände führen kann. Um denselben Stimulationserfolg zu erzielen, muss dann infolge dieser Adaptation mit höherer Reizamplitude stimuliert werden. Je größer die Reizamplitude ist, desto größer ist die Wahrscheinlichkeit, dass es infolge der Reizung von Nachbararealen zu Nebenwirkungen - wie Dysarthrie (Sprechstörungen), Dysästhesie (zum Teil sehr schmerzhafte Missempfindungen), zerebelläre Ataxie (Unfähigkeit, ohne fremde Hilfe sicher zu stehen) oder Schizophrenie artigen Symptomen etc. - kommt. Diese Nebenwirkungen können vom Patienten nicht toleriert werden. Die Behandlung verliert daher in diesen Fällen nach wenigen Jahren ihre Wirksamkeit.

Bei anderen Stimulationsmethoden, wie sie beispielsweise in DE 102 11 766 A1 beschrieben sind, wurde vorgeschlagen, dass bedarfsgesteuert Reize im jeweiligen Zielgebiet appliziert werden. Das Ziel dieser Verfahren/dieser Vorrichtungen ist es, das krankhaft synchrone Feuern nicht - wie bei der Standard-Tiefenstimulation - einfach zu unterdrücken, sondern näher an das physiologische, unkorrelierte Feuermuster heran zu bringen. Hierdurch soll einerseits der Stromverbrauch vermindert werden und andererseits durch die bedarfsgesteuerte Stimulation der Energieeintrag in das Gewebe im Vergleich zur Standard-Tiefenstimülation reduziert werden.

Weitere Stimulationstechniken sind aus der Schrift WO 2003/077985 A sowie der Veröffentlichung "Desynchronizing double-pulse phase resetting and application to deep brain stimulation" von P. Tass, erschienen in Biological Cybernetics, Band 85, Nr. 5, 2001, Seiten 343 bis 354, bekannt.

Die Merkmale des Oberbegriffs des Anspruchs 1 sind aus diegen Stand der Technik bekannt.

Die oben erwähnten Stimulationsmethoden wenden als Stimulationssignale Einzelpulse, Hochfrequenz- und Niederfrequenzpulszüge an, die entweder die eigene Dynamik der stimulierten Neuronen unterdrücken oder die zu desynchronisierende Neuronenpopulation durch einen Phasenreset in einen N-Cluster Zustand bringen. Die Stimulationspulszüge werden ohne Verwendung der eigenen Dynamik der zu desynchronisierenden Neuronenpopulation konstruiert und sind, in diesem Sinn, fremde und unphysiologische Signale für die zu desynchronisierende Neuronenpopulation. Um die krankhafte Symptomatik zu unterdrücken, müssen die Stimulationspulszüge mit hoher Intensität appliziert werden, was die Adaption der zu desynchronisierenden Neuronenpopulation an die unphysiologischen Reize und eventuelle Nebenwirkungen erwarten lässt.

Gegenstand der Erfindung ist daher eine Vorrichtung zur Desynchronisation von neuronaler Hirnaktivität zu schaffen, mit der Patienten mit krankhaft synchronisierter Hirnaktivität mild und effizient behandelt werden können. Hierbei soll eine Adaptation an einen unphysiologischen Dauerreiz unterbunden werden. Es sollen langwierige Kalibrierungsvorgänge verhindert werden und die Stimulation soll auch dann erfolgreich sein, wenn die Hauptfrequenz-Komponente der pathologisch rhythmischen Aktivität starken Schwankungen unterliegt. Des weiteren soll die Vorrichtung eine dauerhafte Desynchronisation erreichen, transiente, stimulationsbedingte unphysiologische Zustände sollen weitestgehend vermieden werden. Die erfindungsgemäße Vorrichtung bedarf keiner zusätzlichen Bedarfssteuerung, die wie in Abschnitt 6.3 beschrieben optional hinzugefügt werden kann, daher ist sie leicht realisierbar und es werden nur geringe Ansprüche an die Komplexität der Steuerelektronik und damit auch an den Stromverbrauch gestellt. Die erfindungsgemäße Stimulationsvorrichtung soll Strom sparend funktionieren, so dass die Batterien des in den Patienten implantierten Stimulators seltener operativ ausgetauscht werden müssen.

Ausgehend vom Oberbegriff des Anspruchs 1 wird die Aufgabe erfindungsgemäß gelöst durch die im kennzeichnenden Teil des Anspruchs 1 angegebenen Merkmale. Unter Verwendung der gemessenen und bearbeiteten Aktivität der zu desynchronisierenden Neuronenpopulation als Feedback-Stimulationssignal, siehe Abschnitt 3, wird die Aufgabe dadurch gelöst, dass die Neuronen von mindestens zwei Teilbereichen eines Hirnareals oder mindestens zwei funktionell zusammengehörigen Hirnarealen mit mindestens zwei Elektroden durch die Stimulation mit Einzelreizen unterschiedlicher Zeitverzögerungen in ihrer Aktivität jeweils derart beeinflusst werden, dass es überraschenderweise zu einer vollständigen Desynchronisation der stimulierten Neuronenpopulation kommt wodurch bei einer erkrankten Person die Symptomatik unterdrückt wird. Hierzu umfasst die erfindungsgemäße Vorrichtung eine Steuerung 4, die das Messsignal des Sensors 3 oder der Sensoren 3 aufnimmt und aus diesem Signal mindestens zwei Stimulationssignale generiert und an die Elektroden 2 weiter gibt.

Die erfindungsgemäße Vorrichtung arbeitet Strom sparend, so dass im Patienten implantierte Batterien seltener ausgetauscht werden müssen.

Die erfindungsgemäße Vorrichtung ermöglicht es, den mit der desynchronisierenden Stimulation intraoperativ erzielten Effekt zur Auswahl des am besten geeigneten Zielpunkts für die Tiefenelektrode zu nutzen. Hierzu wird während der Implantation der Tiefenelektrode im Bereich des anatomisch vorberechneten Zielpunkts in mm-Schritten vorangehend eine Testreizung mit der erfindungsgemäßen Vorrichtung durchgeführt. Der Zielpunkt, bei welchem sich der beste therapeutische Effekt erzielen lässt, wird als Zielpunkt für die dauerhafte Implantation gewählt. Außerdem können neben den oben genannten Erkrankungen, die häufig anhaltende pathologisch synchrone Aktivität mit relativ konstanter Frequenz aufweisen, auch Erkrankungen behandelt werden, bei denen es nur intermittent (kurzzeitig auftretend) zu pathologisch synchroner Aktivität kommt. Eine Hauptindikation ist dabei die Behandlung von medikamentös nicht (mehr) behandelbaren Epileptikern. Die erfindungsgemäße Vorrichtung kann beispielsweise bei den Krankheiten Morbus Parkinson, essentieller Tremor, Dystonie, Epilepsie und Zwangserkrankungen eine Desynchronisation bewirken.

Vorteilhafte Weiterbildungen der Erfindung sind in den Unteransprüchen angegeben.

Die Figuren zeigen beispielhafte Ausführungsformen der Erfindung:

Es zeigt:

Fig.1: Eine erfindungsgemäße Vorrichtung

Fig.2a: Zeitgang des Synchronisationsmaßes während eines Stimulationsintervalls. Kleine (große) Werte entsprechen geringer (starker) Synchronisation. Die Stimulation beginnt zum Zeitpunkt 2 Sekunden und wird zum Zeitpunkt 25 Sekunden beendet.

Fig.2b: Zeitgang der über Sensor 3 gemessenen neuronalen Aktivität der Nervenzellen während der Stimulation von Figur 2.

Fig.2c: Zeitgang des über eine Elektrode 2 applizierten Einzelreizes während der Stimulation von Figur 2.

Fig.3: Beispiel für eine Applikation eines Stimulationsmusters über 4 Elektroden mit vier verschiedenen Zeitverzögerungen.

Fig.4: Beispiel für eine Reizapplikation mit 4 Elektroden und zwei verschiedenen Zeitverzögerungen und unterschiedlicher Polarität.

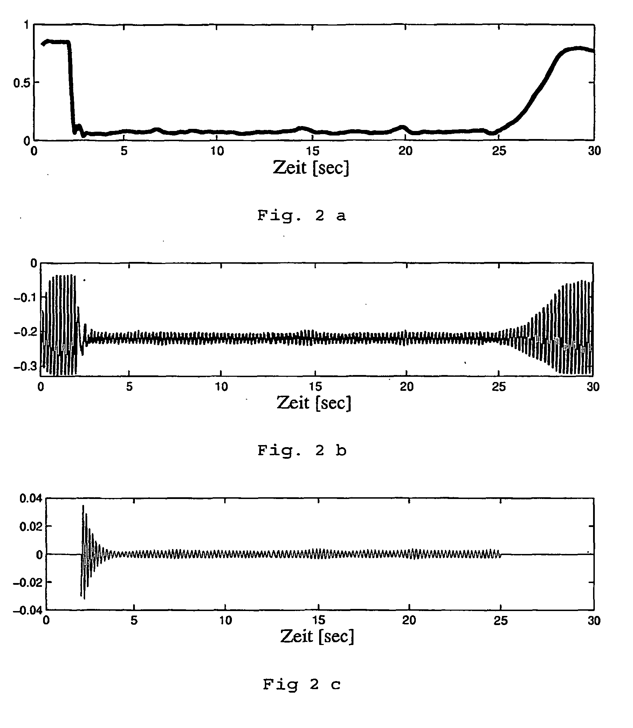

In den Figuren 2a, b und c bezeichnen die Abszissen die Zeitachsen in Sekunden, während auf den Ordinaten das Synchronisationsmaß (Fig.2a) bzw. die gemessene neuronale Aktivität (Fig.2b) bzw. ein beispielhafter Einzelreiz (Fig.2c) jeweils in willkürlichen Einheiten aufgetragen sind. Die über Sensor 3 gemessene neuronale Aktivität (Fig.2b) dient als Grundlage zur Erstellung der Einzelreize. Die über Sensor 3 gemessene neuronale Aktivität (Fig.2b) dient als Steuer-Signal für die Reizapplikation.

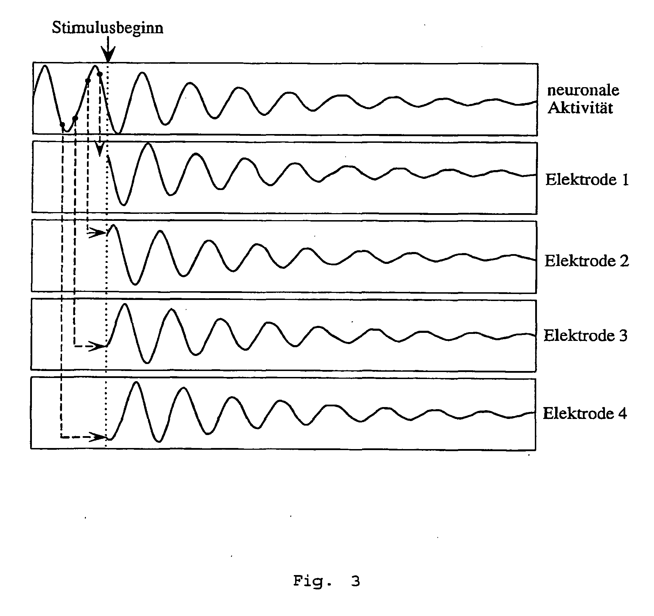

In Figur 3 ist die Abszisse die Zeitachse in Sekunden, während auf der Ordinate die gemessene neuronale Aktivität in willkürlichen Einheiten und die Einzelreize, zum Beispiel im Sinne des applizierten Stroms, in willkürlichen Einheiten dargestellt ist. Über die vier Elektroden 2 wird dasselbe Stimulationsmuster mit derselben Polarität appliziert, jedoch mit vier unterschiedlichen, beispielsweise äquidistanten Zeitverzögerungen.

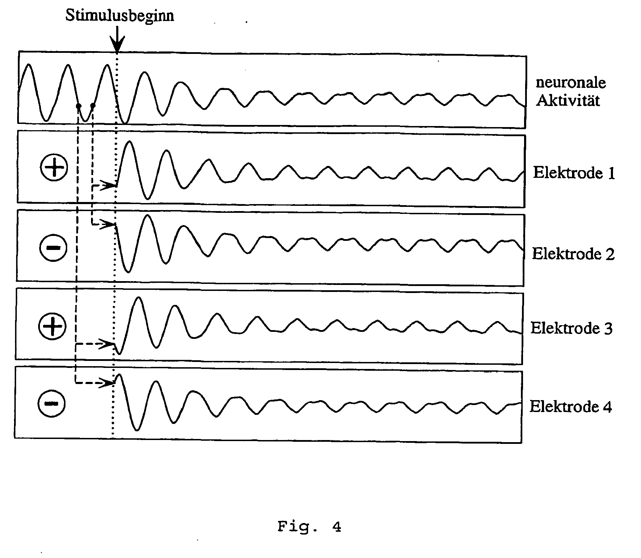

In Figur 4 ist die Abszisse die Zeitachse in Sekunden, während auf der Ordinate die gemessene neuronale Aktivität in willkürlichen Einheiten und die Einzelreize, zum Beispiel im Sinne des applizierten Stroms, in willkürlichen Einheiten dargestellt ist. Alternativ zur Veränderung der Zeitverzögerungen kann auch die Polarität der Einzelreize verändert werden. Beispielsweise kann über die ersten beiden Elektroden 2 ein Stimulationsreiz mit gleicher Zeitverzögerung, aber mit unterschiedlicher Polarität appliziert werden. Entsprechend wird über die dritte und vierte Elektrode 2 ein Stimulationsreiz mit einer anderen Zeitverzögerung aber unterschiedlicher Polarität appliziert. Die jeweilige Polarität der Einzelreize ist durch die Symbole "+" und "-" gekennzeichnet.

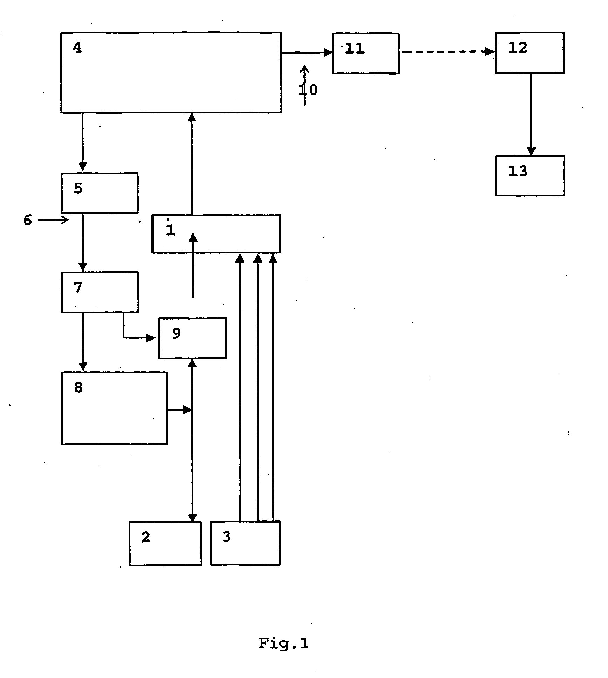

Die Vorrichtung gemäß Figur 1 umfasst einen Trennverstärker 1, an den mindestens zwei Elektroden 2 sowie mindestens einen Sensor 3 zur Erfassung von physiologischen Messsignalen angeschlossen sind. Der Trennverstärker steht weiterhin mit einer Einheit 4 zur Signalverarbeitung und Steuerung in Verbindung, welche an einen optischen Sender für die Stimulation 5 angeschlossen ist. Der optische Sender 5 ist über Lichtwellenleiter 6 mit einem optischen Empfänger 7 verbunden, welcher mit einer Stimulatoreinheit 8 zur Signalerzeugung in Verbindung steht. Die Stimulatoreinheit 8 für die Signalerzeugung steht mit mindestens zwei Elektroden 2 in Verbindung. Am Eingangsbereich der Elektroden 2 in den Trennverstärker 1 befindet sich ein Relais 9 oder Transistor. Die Einheit 4 steht über eine Leitung 10 mit einem Telemetriesender 11 in Verbindung, welcher mit einem Telemetrieempfänger 12 in Verbindung steht, der sich außerhalb des zu implantierenden Geräts befindet und an den ein Mittel zur Visualisierung, Verarbeitung und Speicherung der Daten 13 angeschlossen ist. Als Sensoren 3 können beispielsweise epikortikale Elektroden, Tiefenelektroden, Hirnelektroden oder periphere Elektroden eingesetzt werden.

Bei den Elektroden 2 handelt es sich um jeweils mindestens zwei Drähte, an deren Enden eine Potentialdifferenz zum Zwecke der Stimulation angelegt wird. Es kann sich dabei um Makro- oder Mikroelektroden handeln. Alternativ kann es sich bei den Elektroden 2 auch um jeweils einzelne Drähte handeln. In diesem Fall wird zum Zwecke der Stimulation jeweils eine Potentialdifferenz zwischen einem einzelnen Draht und dem metallischen Teil des Gehäuses des Generators angelegt. Zusätzlich, aber nicht zwingend, kann über die Elektroden 2 eine Potentialdifferenz gemessen werden, um eine pathologische Aktivität festzustellen. In einer weiteren Ausführungsform können die Elektroden 2 auch aus mehr als zwei einzelnen Drähten bestehen, die sowohl für die Ermittlung eines Messsignals im Gehirn, als auch für die Stimulation herangezogen werden können. Beispielsweise können vier Drähte in einem Leiterkabel untergebracht sein, wobei zwischen verschiedenen Enden eine Potentialdifferenz angelegt oder gemessen werden kann. Hierdurch lässt sich die Größe des abgeleiteten bzw. stimulierten Zielgebietes variieren. Die Anzahl der Drähte, aus welchen sich die Elektrode aufbaut, ist nach oberen Werten hin lediglich durch die damit verbundene Dicke des in das Gehirn einzuführenden Kabels begrenzt, so dass möglichst wenig Hirnmaterial beschädigt werden soll. Handelsübliche Elektroden umfassen vier Drähte, es können jedoch auch fünf, sechs oder mehr Drähte, aber auch nur drei Drähte umfasst sein.

Für den Fall, dass die Elektroden 2 mehr als zwei Drähte umfassen, können mindestens einer dieser Drähte auch als Sensor 3 fungieren, so dass in diesem Fall eine Ausführungsform vorliegt, bei der die Elektroden 2 und der Sensor 3 in einem einzigen Bauteil vereint sind. Die Drähte der Elektroden 2 können unterschiedliche Längen haben, so dass sie in verschiedene Hirntiefen eindringen können. Bestehen die Elektroden 2 aus n Drähten, wobei n eine ganze Zahl ist, so kann eine Stimulation über mindestens ein Paar von Drähten erfolgen, wobei bei der Paarbildung jede Unterkombination von Drähten möglich ist. Neben diesem Bauteil können auch zusätzlich nicht mit den Elektroden 2 baulich vereinte Sensoren 3 vorhanden sein.

Beispielhaft und anschaulich gesprochen wird durch die erfindungsgemäße Vorrichtung in einem ersten Schritt mittels der Sensoren die neuronale Aktivität gemessen. In einem zweiten Schritt werden die Stimulationssignale durch zeitliche Verzögerung und gegebenenfalls durch weitere Bearbeitung der neuronalen Aktivität generiert. Über mindestens zwei implantierte Elektroden werden diese Stimulationssignale sodann in einem dritten Arbeitsschritt mit vorzugsweise unterschiedlichen Zeitverzögerungen zur Stimulation verwendet. Als Folge dieser Stimulation tritt im stimulierten Gewebe eine Desynchronisation ein. Details der Funktionsweise der erfindungsgemäßen Vorrichtung sind in Abschnitt 1 erläutert.

Wie in Abschnitt 6 beschrieben kann die erfindungsgemäße Vorrichtung in verschiedenen Ausführungsformen der zeitlichen Steuerung der Reizapplikation realisiert werden. Die Varianten der zeitlichen Steuerung der Reizapplikation sind permanente, wiederkehrende und bedarfsgesteuerte Reizapplikation.

Die erfindungsgemäße permanente Reizapplikation ist eine einfache Ausführungsform der erfindungsgemäßen Vorrichtung, die ohne zusätzliche Bedarfssteuerung arbeitet und permanent, wie in Abschnitt 6.1 beschrieben, Reize appliziert. Somit stellt die permanente Reizapplikation eine leicht zu realisierende Ausführungsform der erfindungsgemäßen Vorrichtung dar. Gleichzeitig erfolgt aufgrund der in Abschnitt 5 beschriebenen erfindungsgemäßen selbstregulierenden Bedarfssteuerung eine gute desynchronisierende Wirkung der permanenten Stimulation bei geringem Energieeintrag in die Zielpopulation.

Bei der erfindungsgemäßen wiederkehrenden Reizapplikation verfügt die erfindungsgemäße Vorrichtung über eine Steuerung, die so programmiert ist, dass sie an den Elektroden 2 eine Applikation der Stimulationssignale nur während bestimmter Zeitintervalle vornimmt. Außerhalb dieser Zeitintervalle erfolgt keine Stimulation. Die Steuereinheit 4 ist daher so programmiert, dass in der Ausführungsform der in Abschnitt 6.2 beschriebenen wiederkehrenden Stimulation zu von Steuereinheit 4 bestimmten, vorzugsweise periodisch aufeinander folgenden Zeitpunkten ein Stimulationssignal mit einer von Steuereinheit 4 berechneten Dauer generiert und an die Elektroden 2 abgegeben wird. Wie im Fall der permanenten Reizapplikation findet die selbstregulierende Bedarfssteuerung der Amplitude des Stimulationssignals auch bei der wiederkehrenden Reizapplikation statt.

Bei der erfindungsgemäßen bedarfsgesteuerten Reizapplikation verfügt die erfindungsgemäße Vorrichtung über eine zusätzliche Bedarfssteuerung. Dafür ist die erfindungsgemäße Vorrichtung vorzugsweise mit Mitteln ausgestattet, welche die Signale der Elektroden 2 und/oder der Sensoren 3 als pathologisch erkennen und im Falle des Vorliegens eines pathologischen Musters über die Elektroden 2 Reize abgeben, die bewirken dass die pathologische neuronale Aktivität in den von den einzelnen Elektroden 2 stimulierten Subpopulationen eine Desynchronisation erfährt und somit der natürlichen, physiologischen Aktivität näher kommt. Die pathologische Aktivität unterscheidet sich von der gesunden Aktivität durch eine charakteristische Veränderung ihres Musters und/oder ihrer Amplitude und/oder ihres Frequenzgehalts. Die Mittel zum Erkennen des pathologischen Musters sind dabei ein Rechner, der die gemessenen Signale der Elektroden 2 und/oder des Sensors 3 verarbeitet und mit im Rechner gespeicherten Daten vergleicht. Der Rechner verfügt über einen Datenträger, welcher Daten speichert. Diese können im Rahmen der Kalibrierung und/oder Steuerung gemäß der Abschnitte 6 und 7 benutzt werden. Die Steuereinheit 4 kann beispielsweise einen Chip oder eine andere elektronische Vorrichtung mit vergleichbarer Rechenleistung umfassen.

In Abhängigkeit vom Auftreten und der Ausprägung pathologischer Merkmale in der bearbeiteten neuronalen Aktivität, wird in der Ausführungsform der in Abschnitt 6.3 beschriebenen bedarfsgesteuerten Reizapplikation ein Reizsignal an die Elektroden 2 abgegeben, so dass eine Stimulation des Hirngewebes erfolgt. Die erfindungsgemäße Vorrichtung verfügt über Mittel zum Erkennen des Auftretens und/oder der Ausprägung der pathologischen Merkmale in der über Sensor 3 gemessenen neuronalen Aktivität. Die Steuereinheit 4 ist so programmiert, dass in der Ausführungsform der in Abschnitt 6.3 beschriebenen bedarfsgesteuerten Reizapplikation zu einem von Steuereinheit 4 ein Stimulationssignal generiert und an die Elektroden 2 abgegeben wird. Insgesamt sollen alle für die jeweilige Verfahrensweise der erfindungsgemäßen Vorrichtung relevanten Parameter für die Art und Stärke der Stimuli, als auch deren zeitliche Verzögerungen sowie Information zur elektrodenspezifischen Applikation als auch die für die bedarfsgesteuerte Funktionsweisen relevanten, über Sensor 3 ermittelten Messwerte bzw. daraus abgeleiteten Parameter abgespeichert werden.

Die Steuereinheit 4 steuert die Elektroden 2 vorzugsweise in folgender Weise an: Die Steuerdaten werden von der Steuereinheit 4 an einen optischen Sender für die Stimulation 5 weitergegeben, welcher über den Lichtleiter 6 den optischen Empfänger 7 ansteuert. Durch das optische Einkoppeln von Steuersignalen in den optischen Empfänger 7, wird eine galvanische Entkopplung der Stimulationssteuerung von den Elektroden 2 bewirkt. Dies bedeutet, dass eine Einstreuung von Störsignalen von der Einheit zur Signalverarbeitung und Steuerung 4 in die Elektroden 2 verhindert wird. Als optischer Empfänger 7 kommt beispielsweise eine Photozelle in Betracht. Der optische Empfänger 7 gibt die über den optischen Sender für die Stimulation 5 eingegebenen Signale an die Stimulatoreinheit 8 weiter. Über die Stimulatoreinheit 8 werden dann gezielte Stimuli über die Elektroden 2 an die Zielregion im Gehirn weitergegeben. Für den Fall, dass über die Elektroden 2 auch gemessen wird, wird ausgehend vom optischen Sender für die Stimulation 5 über den optischen Empfänger 7 auch ein Relais 9 angesteuert, wodurch die Einstreuung von Störsignalen verhindert wird. Das Relais 9 oder der Transistor stellt sicher, dass die neuronale Aktivität unmittelbar nach jedem Stimulus wieder gemessen werden kann, ohne dass der Trennverstärker übersteuert. Die galvanische Entkopplung muss nicht zwingend durch eine optische Einkopplung der Steuersignale erfolgen, vielmehr können auch andere alternative Steuerungen verwendet werden. Diese können beispielsweise akustische Einkopplungen zum Beispiel im Ultraschallbereich sein. Eine störungsfreie Steuerung kann auch beispielsweise unter Zuhilfenahme geeigneter analoger oder digitaler Filter realisiert werden.

Weiterhin steht die erfindungsgemäße Vorrichtung vorzugsweise mit Mitteln zur Visualisierung und Verarbeitung der Signale sowie zur Datensicherung 13 über den Telemetrieempfänger 12 in Verbindung. Dabei kann die Einheit 13 über die unten erwähnten Verfahren zur Datenanalyse verfügen.

Weiterhin kann die erfindungsgemäße Vorrichtung über den Telemetrieempfänger 13 mit einer zusätzlichen Referenzdatenbank in Verbindung stehen, um beispielsweise den ordnungsgemäßen Betrieb des Gerätes zu überwachen und ggf. die in Abschnitt 7.2 beschriebenen Steuermechanismen durch Modifikation der Parameter effizienter auszugestalten.

In Abschnitt 1 wird der Mechanismus der Stimulation ausführlich erläutert. Eine Definition der wichtigsten Begriffe ist in Abschnitt 2 zu finden. Die Arbeitsschritte von der Messung der neuronalen Aktivität über deren Bearbeitung bis zu der Generierung der Gesamtreize ist in Abschnitt 3 erläutert. Die räumliche Anordnung der Elektroden und Sensoren ist Gegenstand von Abschnitt 4. Abschnitt 5 befasst sich mit der selbstregulierenden Bedarfssteuerung der Stimulationsamplitude. Die Steuerung der Reizapplikation und die Kalibrierung und Anpassung der Stimulationsparameter wird in den Abschnitten 6 und 7 beschrieben. Die Vorteile der erfindungsgemäßen Vorrichtung sind in Abschnitt 8 angeführt.

## 1 Mechanismus der Stimulation

Ziel der Stimulation ist es, einer krankheitsbedingt vorhandenen Synchronisation in einer Nervenzellpopulation durch Desynchronisation entgegenzuwirken.

Dies geschieht, in dem an mindestens zwei Stellen Reize appliziert werden, die dadurch generiert werden, dass neuronale Aktivität gemessen und nach einem gegebenenfalls vorhandenen Bearbeitungsschritt in ein Stimulationssignal umgewandelt und vorzugsweise zeitlich verzögert appliziert wird, so dass sich überraschenderweise eine Desynchronisation einstellt. Die desynchronisierende Wirkung der Stimulation wird durch die krankhaft gesteigerte Interaktion zwischen den Neuronen und die Abnahme der Stimulationswirkung mit dem Abstand des stimulierten Gewebes zur Elektrode unterstützt. Man nutzt anschaulich gesprochen die Energie des zu beeinflussenden Systems aus, um mit minimalem Eingriff einen therapeutischen Effekt zu erzielen. Mit der erfindungsgemäßen Vorrichtung wird die zu desynchronisierende Nervenzellpopulation unmittelbar in einen desynchronisierten Zustand gebracht. Der gewünschte Zustand, das heißt die komplette Desynchronisation, stellt sich typischerweise, während weniger Perioden der neuronalen Aktivität, häufig in weniger als einer Periode ein.

Typischerweise besteht die Notwendigkeit permanenter oder wiederkehrender Stimulation, da die zu desynchronisierende Nervenzellpopulation nach Ausschalten der Stimulation erfahrungsgemäß wieder resynchronisiert. Da die Stimulation direkt mit der neuronalen Aktivität des Zielareals oder eines zugeordneten Areals zusammenhängt, wird die Stimulationsamplitude nach erfolgreicher Desynchronisation automatisch minimiert. Dies wird ermöglicht, dadurch dass als Stimulationsreiz das Feedback-Stimulationssignal, das heißt die bearbeitete neuronale Aktivität, benutzt wird, d.h. das Ausmaß der Synchronisation steuert permanent die Stärke der Stimulation. Dieser Prozess funktioniert für einen großen Bereich der modifizierbaren Stimulationsparameter, wie beispielsweise Stimulationsperiode T, die Zeitverzögerung und die Intensität, benötigt keine aufwendige Kalibrierung und verfügt über eine große Fehlertoleranz. Des weiteren wird der Energieeintrag in das zu desynchronisierende Gewebe aufgrund des direkten Zusammenhangs zwischen neuronaler Aktivität und Stimulationsmuster minimiert, was geringere Nebenwirkungen erwarten lässt.

Im Folgenden soll die erfindungsgemäße Vorrichtung und deren Funktionieren beispielhaft erläutert werden.

Die erfindungsgemäße Vorrichtung und die Steuerung sind mit Mitteln ausgestattet, die alle Schritte des erfindungsgemäßen Behandlungsverfahrens durchführen können. Mit den offenbarten Verfahrensschritten sollen daher implizit auch Mittel zur Durchführung des Verfahrensschrittes offenbart sein. Die Verfahrensschritte stellen somit auch gleichzeitig die funktionalisierten Vorrichtungsmerkmale dar.

Erfindungsgemäß werden die Elektroden in die Hirnregion eingebracht, welche für die Ausbildung des Krankheitsbildes verantwortlich ist. Entweder direkt in das Gebiet oder in eine oder mehrere mit diesem Gebiet verbundenen Nervenzellpopulationen oder Nervenfaserbündel werden erfindungsgemäß mindestens zwei bevorzugt vier aber auch drei oder mehr Elektroden eingebracht. Die Anzahl der Elektroden ist lediglich dadurch begrenzt, dass keine beliebig hohe Dichte an Elektroden in einer Hirnregion vorhanden sein soll, damit das Gewebe nicht unnötig beschädigt wird und vor allem das Blutungsrisiko beim Einführen der Elektroden vermindert wird. Jedenfalls soll die Anzahl der in die Region eingebrachten Elektroden $N$ sein, mit $N \geq 2$.

Jede Elektrode gibt dabei in ihrer Umgebung ein Signal ab, welches entweder direkt in ihrem Umfeld oder über ein Nervenfaserbündel fortgeleitet in einem anderen Areal eine Desynchronisation bewirkt. Um eine Desynchronisation herbeizuführen, wird die gemessene und bearbeitete, in jedem Fall zeitlich verzögerte, neuronale Aktivität, siehe Abschnitt 3, als Stimulationsreiz verwendet. Die erfindungsgemäße Vorrichtung verfügt daher über eine Steuerung, welche mindestens zwei Elektroden 2 so ansteuert, dass sie in ihrem näheren Umfeld und/oder durch Weiterleitung der Stimulation über ein Faserbündel in einem anderen Gehirnareal eine Desynchronisation bewirken.

Erfindungsgemäß werden $N$ Elektroden mit $N \geq 2$ vorzugsweise so angesteuert, dass eine zeitliche Verschiebung der einzelnen Elektrodensignale um $T/N$ vorliegt, sofern die stimulierenden Elektroden 2 sich in dem zu desynchronisierenden Areal befinden. $T$ ist hierbei, wie unten beschrieben, die Periode der rhythmischen, zu desynchronisierenden neuronalen Aktivität. Falls sich mindestens eine der stimulierenden Elektroden 2 nicht in dem zu desynchronisierenden Areal befindet, ist bei der Ansteuerung einer solchen Elektrode 2 die Laufzeit zwischen dem Reizort, und dem Ort der hierdurch beeinflussten Neuronenpopulation zu berücksichtigen. Dies wird in Abschnitt 7.3 beschrieben. Dem gemäß verfügt die erfindungsgemäße Vorrichtung über eine Steuerung, die bei $N$ Elektroden vorzugsweise ein um im wesentlichen ein $N$-tel der Periode der zu desynchronisierenden Aktivität zeitverschobenes Stimulationssignal erzeugt. Die zeitliche Verschiebung ist dabei vorzugsweise im wesentlichen äquidistant.

Überraschenderweise wird bei dieser äquidistanten zeitlichen Verschiebung der von den $N$ Elektroden 2 jeweils beeinflussten Neuronenpopulation nicht einfach nur eine Aufteilung der Neuronenpopulation in $N$ jeweils für sich synchronisierte Teilpopulationen erzeugt. Vielmehr kommt es bei dieser Stimulation überraschenderweise zu einer Desynchronisation der gesamten, zu desynchronisierenden Neuronenpopulation, was mit einer Unterdrückung der pathologischen Symptome einhergeht. Befindet sich mindestens eine Elektrode 2 außerhalb des zu desynchronisierenden Areals, so müssen Effekte der indirekten Stimulation, wie sie in Abschnitt 7.3 beschrieben sind, berücksichtigt werden.

Mit dem neuen Verfahren/der neuen Vorrichtung wird die Desynchronisation im Vergleich zum oben genannten Stand der Technik qualitativ anders erzielt. Anstatt den krankhaft synchronen Nervenzellverband gezielt in einer vulnerablen Phase seines Rhythmus zu treffen, wird der betroffene Nervenzellverband einfach an mehreren Orten zeitlich koordiniert, siehe Abschnitt 3.3, in einer Weise stimuliert, dass eine Desynchronisation auftritt.

Hierbei wird an den einzelnen Reizorten die gemäß Abschnitt 3.2 bearbeitete neuronale Aktivität verwendet. Es muss an mindestens zwei, vorzugsweise mehr als zwei Reizorten stimuliert werden. Die überraschenderweise eintretende Desynchronisation wird durch die krankhaft gesteigerte Interaktion zwischen den Neuronen und durch die mit der Entfernung zwischen Stimulationsort und zu stimulierenden Neuron abnehmende Stimulationswirkung unterstützt. Man nutzt hierbei einen Wirkmechanismus aus, welcher für die krankhafte Synchronisation verantwortlich ist. Man nutzt anschaulich gesprochen die Energie des zu beeinflussenden Systems aus, um mit minimalem Eingriff einen therapeutischen Effekt zu erzielen. Die besten Ergebnisse werden erzielt, wenn Gesamtreize mit im wesentlichen äquidistanten Zeitverzögerungen verwendet werden. Es werden aber auch noch Behandlungserfolge erzielt, wenn die Zeitverzögerungen der über die Elektroden 2 abgegebenen Reize nicht äquidistant sind. In so einem Fall wird zumindest eine teilweise Desynchronisation herbeigeführt. Die Behandlungsergebnisse werden aber umso besser, je mehr die gewählten Zeitverzögerungen äquidistanten Zeitverzögerungen nahe kommen.

## 2 Definition der Begriffe

Zielpopulation:

Im Folgenden wird unter der Zielpopulation die unmittelbar durch eine implantierte Stimulationselektrode stimulierte Nervenzellpopulation verstanden.

Eine Zielpopulation wird durch eine in ihr oder nahe bei ihr implantierte Elektrode direkt stimuliert. Die Nervenzellpopulation, welche krankhaft synchron aktiv ist, wird als zu desynchronisierendes Areal oder als zu desynchronisierende Nervenzellpopulation oder als zu desynchronisierende Neuronenpopulation bezeichnet. Das zu desynchronisierende Areal ist nicht an anatomische Grenzen gebunden. Vielmehr kann darunter auch mindestens eine Komponente, bestehend aus der Gruppe

- mindestens ein Teil von mindestens einem anatomischen Areal,
- mindestens ein vollständiges anatomisches Areal, verstanden werden.

Das zu desynchronisierende Areal kann entweder direkt oder indirekt stimuliert werden.

Direkte Stimulation:

In diesem Fall befindet sich die Stimulationselektrode 2 unmittelbar in dem zu desynchronisierenden Areal. Diese Elektrode 2 beeinflusst dabei die Zielpopulation, welche sich in dem zu desynchronisierenden Areal befindet.

Indirekte Stimulation:

In diesem Fall wird das zu desynchronisierende Areal mittels der Stimulationselektrode 2 nicht direkt stimuliert. Vielmehr wird über die Elektrode 2 eine Zielpopulation oder ein Faserbündel, welche mit dem zu desynchronisierenden Areal funktionell eng verbunden sind, stimuliert. Hierbei wird der Stimulationseffekt auf das zu desynchronisierende Areal vorzugsweise über anatomische Verbindungen fortgeleitet. Für die indirekte Stimulation soll als Oberbegriff für Zielpopulation und Faserbündel der Begriff Zielareal eingeführt werden. Von dem Begriff Zielareal sollen im Folgenden die mit dem zu desynchronisierenden Areal funktionell eng verbundene Neuronenpopulation und das verbindende Faserbündel verstanden werden, die unmittelbar durch implantierte Elektroden 2 stimuliert werden.

Neuronale Aktivität:

Die Beschreibung des Mechanismus der erfindungsgemäßen Vorrichtung ist wesentlich gestützt auf dem Begriff der neuronalen Aktivität. Die neuronale Aktivität der zu desynchronisierenden Neuronenpopulation und/oder einer damit eng verbundenen Neuronenpopulation wird gemessen, gespeichert und gemäß Abschnitt 3.2 bearbeitet und als Stimulationssignal verwendet, wodurch die erfindungsgemäße selbstregulierende Bedarfssteuerung realisiert wird. Im Folgenden wird unter der gemessenen neuronalen Aktivität der zu desynchronisierenden Neuronenpopulation ein Signal verstanden, welches die zeitliche Entwicklung der Aktivität der zu desynchronisierenden Neuronenpopulation wiedergibt. Beispielsweise können lokale Feldpotentiale die zeitliche Entwicklung der Aktivität der zu desynchronisierenden Neuronenpopulation wiedergeben. Die neuronale Aktivität kann vorzugsweise direkt im zu desynchronisierenden Areal gemessen werden, es kann aber auch eine der neuronalen Aktivität des zu desynchronisierenden Areals zugeordnete Aktivität beispielsweise eines anderen Hirnareals, hier zum Beispiel dem Motorkortex, oder die Aktivität einer durch das zu desynchronisierende Areal kontrollierte Muskelgruppe gemessen werden. In einer weiteren Ausführungsform der erfindungsgemäßen Vorrichtung können neuronale Aktivitäten, an verschiednen Orten gemessen und kombiniert werden, um eine hinreichende Repräsentation der neuronalen Aktivität der zu desynchronisierenden Neuronenpopulation zu erhalten. Auch diese der neuronalen Aktivität des zu desynchronisierenden Areals zugeordnete Größen werden im Folgenden als neuronale Aktivität bezeichnet.

Feedback-Stimulationssignal:

Unter dem Feedback-Stimulationssignal wird das Signal verstanden, welches gemäß Abschnitt 3.2 die gemessene und bearbeitete neuronale Aktivität darstellt und als Grundlage für die Einzelreize dient.

Rhythmus:

Unter einem Rhythmus wird die rhythmische, also annähernd periodische neuronale Aktivität verstanden, die sich in Folge einer krankhaft übersteigert synchronen Aktivität von Nervenzellen ergibt. Ein Rhythmus kann kurzzeitig auftretend oder lang anhaltend sein.

Periode:

Ein zentraler Begriff für die erfindungsgemäße Vorrichtung ist die Periode der rhythmischen neuronalen Aktivität, die als zeitliche Referenz für die Applikation der Einzelreize dient. Durch Anpassung der Stimulationsperiode T, wie in Abschnitt 7.2.1 beschrieben, wird vorzugsweise bewirkt, dass die Periode der rhythmischen neuronalen Aktivität mit der Stimulationsperiode T übereinstimmt.

Zeit Verzögerung:

Durch die erfindungsgemäße Vorrichtung werden Signale an die Stimulationselektrode 2 weitergegeben, welche der gemäß Abschnitt 3.1 gemessenen und gegebenenfalls bearbeiteten neuronalen Aktivität zu einem früheren Zeitpunkt entsprechen. Diese zeitliche Verschiebung wird im folgenden Zeitverzögerung genannt und stellt einen wichtigen, im Zusammenhang mit der Periode der rhythmischen neuronalen Aktivität stehenden, Stimulationsparameter dar.

Einzelreiz:

Unter einem Einzelreiz, siehe Abschnitt 3.3, wird im Folgenden sein Stimulationsreiz verstanden, der über eine einzelne Elektrode appliziert wird und in einem Zeitintervall wirkt. Für diese Stimulationsreize wird die gemäß Abschnitt 3.2 bearbeitete neuronale Aktivität verwendet.

Gesamtreiz:

Ein Gesamtreiz ist die Gesamtheit der über die Elektroden applizierten Einzelreize, siehe Abschnitt 3.4.

## 3 Art des Stimulationsreizes

### 3.1 Messung der neuronalen Aktivität

Der zeitliche Verlauf der neuronalen Aktivität des zu desynchronisierenden Areals kann über die Sensoren 3 direkt oder indirekt gemessen werden.

Die Sensoren 3 (siehe Figur 1) befinden sich im Gehirn und/oder außerhalb des Gehirns. Im Gehirn sind sie im zu desynchronisierenden Areal und/oder in mindestens einem anderen, funktionell damit in Verbindung stehenden Areal positioniert. Außerhalb des Gehirns befinden sich die Sensoren 3 an Körperteilen, die mit der krankhaft synchronisierten neuronalen Aktivität in Verbindung stehen, z.B. als Elektroden an einem zitternden Muskel. Die Messsignale der neuronalen und/oder nichtneuronalen, beispielsweise muskulären Aktivität werden in einer Einheit zur Signalverarbeitung 4 verarbeitet und gespeichert. Die Verarbeitung und Speicherung kann dabei permanent und/oder in zeitlich diskreten Abständen erfolgen. Im letzteren Fall werden die Dauer und/oder die Abstände der diskreten Messintervalle durch einen deterministischen und/oder stochastischen Algorithmus bestimmt.

### 3.2 Bearbeitung der neuronalen Messsignale

Die in der Einheit zur Signalverarbeitung 4 gespeicherten Messsignale werden sodann bearbeitet, um als Stimulationssignale zur Verfügung zu stehen. Folgende Bearbeitungsschritte können angewendet werden:

1. Die gemessene neuronale Aktivität kann gefiltert werden, z.B. kann eine Bandpassfilterung der neuronalen Aktivität vorgenommen werden. Die Filterung kann notwenig sein, falls über Sensor 3 neben der krankheitsspezifischen Aktivität zusätzlich noch nicht krankheitsspezifische Aktivität, zum Beispiel aus anderen Neuronenpopulationen, gemessen wird. Da die krankheitsspezifische Aktivität typischerweise in einem Frequenzbereich auftritt, der von dem Frequenzbereich der nichtkrankheitsspezifischen Aktivität verschieden ist, wird in diesem Falle vorzugsweise eine Ermittlung der Aktivität im krankheitsspezifischen Frequenzbereich durchgeführt. Dies wird beispielsweise durch eine Frequenzanalyse realisiert. Ebenso kann es notwendig sein eine Wavelet-Analyse und/oder eine Hilbert-Transformation und/oder eine Filterung in zeitlicher Domäne durchzuführen.
2. Wird über mehrere Sensoren 3 die neuronale Aktivität der zu desynchronisierenden Neuronenpopulation gemessen, so kann eine lineare und/oder nichtlineare Kombination und/oder Transformation zum Beispiel Multiplikation, Summation oder Berechnung einer Funktion, der gemessenen neuronalen Aktivitäten durchgeführt werden.
3. Die gemessene neuronale Aktivität wird zeitlich verzögert. Die hierzu verwendeten Zeitverzögerungen werden in den Abschnitten 3.3 und 3.4 definiert und berücksichtigen auch, gemäß Abschnitt 7.3, die Lage der Stimulationselektroden bezüglich der zu desynchronisierenden Neuronenpopulation. Darüber hinaus können die Zeitverzögerungen vorzugsweise während der Stimulation gemäß Abschnitt 7.2.1 und 7.2.2 angepasst werden.

4. Die gemessene neuronale Aktivität wird verstärkt. Die gemessene neuronale Aktivität ist um einige Größenordnungen geringer, als die Stimulationsamplituden, die erfahrungsgemäß zu einer Stimulationswirkung führt. Daher ist eine Verstärkung, welche während der Stimulation gemäß Abschnitt 7.2.3 angepasst werden kann, durchzuführen.

5. Da Signale mit hohen Gradienten eine große Wirkung auf die neuronale Dynamik haben, wird die gemessene neuronale Aktivität beispielsweise in Form von Pulszügen bzw. Hochfrequenzpulszüge bestehend aus kurzen Rechteckpulsen kodiert. Es können zur Steigerung der Stimulationswirkung auch andere Kodierungsverfahren verwendet werden.

6. Die Polarität der neuronalen Aktivität wird geändert. Dies wurde beispielsweise für den in Abbildung 4 skizzierten Gesamtreiz verwendet.

7. Die maximale Amplitude des Stimulationssignals wird beschränkt.

8. Die gemessene neuronale Aktivität wird dahingehend transformiert, dass Stimulationssignale entstehen, deren Nettoladungseintrag im Wesentlichen Null ist.

Bis auf den Punkt 3 sind die Punkte 1,2 und 4 bis 6 optional anzuwenden.

Die bearbeitete neuronale Aktivität wird durch die Anwendung einer beliebigen Kombination der oben genannten Bearbeitungsschritte ermittelt.

### 3.3 Form der Einzelreize

Unter einem Einzelreiz wird im Folgenden ein Stimulationsreiz verstanden, der über eine einzelne Elektrode appliziert wird und in einem Zeitintervall wirkt. Für diese Stimulationsreize wird das Feedback-Stimulationssignal, das heißt, die gemäß Abschnitt 3.2 bearbeitete neuronale Aktivität, verwendet.

Unter zeitlich koordinierter Stimulation wird hierbei beispielsweise verstanden, dass die Einzelreize über die jeweilige Elektrode 2 mit jeweils geeigneten, vorzugsweise unterschiedlichen Zeitverzögerungen und auch unterschiedlicher Dauer - wie in Abschnitt 3.4 beschrieben - appliziert werden, um zwischen den stimulierten Subpopulationen und innerhalb der Subpopulationen der zu desynchronisierenden Neuronenpopulation eine Desynchronisation zu erzeugen. Die Zeitverzögerungen werden beispielsweise als Bruchteile der Periode der oszillatorischen, zu desynchronisierenden neuronalen Aktivität angegeben und betragen vorzugsweise im wesentlichen ein Vielfaches eines N-tel der Periode, wobei N eine kleine ganze Zahl, zum Beispiel 4, ist. N ist hierbei eine ganze Zahl, vorzugsweise unterhalb von 1000, besonders bevorzugt kleiner als 100 insbesondere kleiner als 10.

Die Zeitverzögerungen der Einzelreize können auch z. B. größer als die Stimulationsperiode T gewählt werden. Hierzu verfügt die erfindungsgemäße Vorrichtung über Mittel, welche die beschriebenen elektrischen Stimulationsreize in der beschriebenen Art applizieren. Die Mittel sind Elektroden 2, eine Steuerung 4, welche Steuersignale an die Elektroden 2 für die Abgabe dieser Reize abgibt. Des weiteren Sensoren 3 und die Einheit zur Signalverarbeitung 4, welche die neuronale Aktivität aufnimmt und für die weitere Verwendung als Stimulationsreize vorbereitet. Als Gesamtreiz werden die über die Elektroden 2 applizierten Einzelreize bezeichnet, welche gemäß des Wirkmechanismus der erfindungsgemäßen Vorrichtung in der zu desynchronisierenden Neuronenpopulation eine Desynchronisation hervorrufen. Beispiele für Gesamtreize sind in den Figuren 3 und 4 gezeigt. Im Rahmen eines Gesamtreizes wird vorzugsweise über jede Elektrode ein Einzelreiz abgegeben.

Bei repetitiver Applikation von Gesamtreizen können die im Rahmen eines Gesamtreizes angesteuerten Elektroden 2 variiert werden. Insbesondere kann die Teilmenge der Elektroden 2, die beim jeweiligen Gesamtreiz angesteuert wird, mittels eines stochastischen und/oder deterministischen Algorithmus ausgewählt werden.

### 3.4 Muster der Gesamtreize

Im Rahmen der Applikation eines Gesamtreizes wird über mindestens zwei Stimulationselektroden 2 vorzugsweise jedoch über jede einzelne Stimulationselektrode 2 ein Einzelreiz appliziert. Vorzugsweise werden Gesamtreize generiert, deren Nettoladungseintrag im wesentlichen Null ist. Der Einzelreiz kann die in Abschnitt 3.3 beschriebenen Formen annehmen.

Die über die verschiedenen Elektroden 2 applizierten Einzelreize können, aber müssen nicht, bezüglich Art und/oder Intensität, zum Beispiel bestimmt durch die Verstärkung, verschieden sein. Zu diesem Zweck verfügt die erfindungsgemäße Vorrichtung über eine Steuerung, die so programmiert ist, dass sie die Art und/oder die Intensität der Einzelreize variieren kann. Die Art und Intensität der Einzelreize wird bestimmt durch die Parameter, die für die, in Abschnitt 3.2 beschriebenen, Bearbeitungsschritte verwendet werden.

Beispielsweise können bei direkter Stimulation über $N$ Elektroden 2 jeweils der gleiche Einzelreiz, in Form der gleichen bearbeiteten neuronalen Aktivität gemäß Abschnitt 3.2, mit einem Unterschied in der Zeitverzögerung von jeweils $T/N$ appliziert werden, wobei $T$ die Stimulationsperiode ist. Beispielsweise kann für $N$=4 mit jeweils um $T/4$ verschobenen Zeitverzögerungen der gleiche kontinuierliche Stimulationsreiz über die erste, zweite, dritte und vierte Elektrode 2 verabreicht werden, wie in Figur 3 dargestellt ist.

Zu diesem Zweck verfügt die erfindungsgemäße Vorrichtung über eine Steuerung, welche so programmiert ist, dass sie $N$ Elektroden 2 mit Einzelreizen ansteuert, deren Zeitverzögerungen im wesentlichen ein Vielfaches von *T/N ist.*

Als weiteres Beispiel können Zeitverzögerungen bei der Reizapplikation durch Wechsel der Polarität der Einzelreize ersetzt werden. Zu diesem Zweck verfügt die erfindungsgemäße Vorrichtung über eine Steuerung, die so programmiert ist, dass sie mindestens eine der Elektroden 2 mit jeweils wechselnder Polarität ansteuern kann. Zum Beispiel können für *N*=4 über die erste und zweite Elektrode 2 und mit einer Zeitverzögerung von *T*/4 über die dritte und vierte Elektrode 2 jeweils ein Paar Einzelreizen gegensätzlicher Polarität appliziert werden, siehe Figur 4.

Alternativ hierzu kann beispielsweise, insbesondere beim in Abschnitt 6.3 beschriebenen bedarfsgesteuerten Reizapplikation, die Zeitverzögerungen und/oder die Polarität und/oder die Applikationsdauer und/oder die Intensität der Einzelreize innerhalb eines Gesamtreizes systematisch oder zufallsgesteuert, das heißt, gemäß einer deterministischen oder stochastischen Regel, variiert werden. Hierzu verfügt die erfindungsgemäße Vorrichtung über eine Steuerung, die so programmiert ist, dass sie die Zeitverzögerungen und/oder die Polarität und/oder die Applikationsdauer und/oder die Intensität der Einzelreize innerhalb eines Gesamtreizes deterministisch und/oder stochastisch ansteuert.

Durch Variation der Zeitverzögerungen und/oder der Polarität und/oder der Applikationsdauer und/oder der Intensität der Einzelreize innerhalb der Gesamtreize kann Adaptationsvorgängen in den Nauronenpopulationen, die eine Erhöhung der Stimulations intensität zum Erreichen der selben therapeutischen Wirkung bewirken, vorgebeugt werden.

## 4 Anzahl und räumliche Anordnung der Elektroden und Sensoren

### 4.1 Anzahl der Stimulationselektroden

Die Anzahl der Elektroden 2 ergibt sich als Kompromiss aus zwei gegenläufigen Bestrebungen: Einerseits sollte die zu desynchronisierende Neuronenpopulation durch die stimulation in möglichst viele funktionelle Subpopulationen aufgeteilt werden. Dies geht umso besser, je mehr Elektroden für die Stimulation verwendet werden. Andererseits soll die Anzahl der zu implantierenden Elektroden möglichst gering gehalten werden, um unnötigen Gewebsschädigungen und vor allem einer Hirnblutung während der Implantation vorzubeugen. Es können mindestens 2 Elektroden eingesetzt werden. Es können auch beispielsweise 3 Elektroden verwendet werden. Besonders bevorzugt ist die Verwendung von 4 Elektroden, da die Desynchronisation bei 4 Elektroden ausgeprägter und länger andauernd wirkt. Mit der Zunahme der Anzahl der Elektroden auf 5 bis zu 100 und mehr wird der Desynchronisationseffekt bezüglich Ausprägung und Dauer verbessert. Die Verwendung von einer größeren Anzahl von Elektroden kann beispielsweise realisiert werden, wenn Mikroelektroden bzw. moderne Neurochiptechnologien verwendet werden.

#### 4.1.1 Ausführungsform für den Fall, dass alle Elektroden 2 in der zu desyncizronisierenden Nervenzellpopulation positioniert sind

Die N Elektroden, wobei N eine ganze Zahl größer als 1 ist, sollen vorzugsweise so angeordnet sein, dass mit jeder einzelnen Elektrode ungefähr ein N-tel der zu desynchronisierenden Nervenzellpopulation stimuliert werden kann. Dies kann mit unterschiedlicher Anzahl der Elektroden und mit unterschiedlicher geometrischer Anordnung der Elektroden zueinander realisiert werden. Es kann beispielsweise eine beliebige, unsymmetrische Anordnung gewählt werden. Bevorzugt werden jedoch im wesentlichen symmetrische Anordnungen, insbesondere in dem Fall einer kleinen Anzahl von Stimulationselektroden, da bei diesen möglicherweise gleiche Abstände zwischen den Elektroden auftreten und somit die stimulationsbedingte funktionelle Aufteilung in gleichwertige Subpopulationen mit dem geringsten Stromeintrag ermöglicht wird. Beispielhaft können die Endpunkte der Elektroden, entlang der Elektroden projiziert, im wesentlichen ein Quadrat ergeben. Es können beispielsweise auch 6 Elektroden verwendet werden. Dabei liegen 4 vorzugsweise im wesentlichen quadratisch angeordnet in einer Ebene, während die anderen beiden im wesentlichen äquidistant senkrecht zu dieser Ebene liegen, wobei ihre Verbindungslinie im wesentlichen die Rotationsachse der 4 quadratisch angeordneten Elektroden bildet. Zur Verwirklichung verschiedener geometrischer Anordnungen können auch die Elektroden mindestens teilweise verschiedene Längen aufweisen. Nach dem Stand der Technik besteht die Möglichkeit mehrere Stimulationselektroden in einer zu implantierenden Stimulationselektrode zu kombinieren, zum Beispiel indem die Stimulationskontakte auf verschiedenen Abständen vom Ende der Elektrode positioniert sind. Dadurch kann der gleiche Stimulationseffekt mit wenigen zu implantierenden Elektroden erreicht werden, was das Auftreten von Hirnschädigungen weiter vermindert.

#### 4.1.2 Ausführungsform für den Fall, dass mindestens eine Elektrode 2 nicht in der zu desynchronisierenden Nervenzellpopulation positioniert ist

Bei dieser Stimulationsform wird in mindestens einem, von dem zu desynchronisierenden Areal, verschiedenen Zielareal stimuliert. Hierbei kann die indirekte Stimulation durch Stimulation einer von der zu desynchronisierenden Nervenzellpopulation verschiedenen Neuronenpopulation und/oder durch Stimulation eines mit der zu desynchronisierenden Nervenzellpopulation verbundenen Faserbündels erfolgen. Dabei kann in einem Zielareal, beziehungsweise in dem zu desynchronisierenden Areal, entweder mindestens eine Elektrode 2 oder eine in Abschnitt 4.1.1 beschriebene Mehrelektroden-Anordnung verwendet werden.

## 4.2 Anzahl der Sensoren

Der Mechanismus der erfindungsgemäßen Vorrichtung besteht im Wesentlichen darin, dass wie in Abschnitt 1 und 3 beschrieben, die gemessenen und bearbeiteten neuronalen Aktivitäten der zu desynchronisierenden Neuronenpopulation wieder als Stimulation appliziert werden. Die Sensoren 3 sind eine der wichtigsten Komponenten der erfindungsgemäßen Vorrichtung und können, wie in Abschnitt 3.1 beschrieben, entweder außerhalb der zu desynchronisierenden Neuronenpopulation oder vorzugsweise direkt in der zu desynchronisierenden Neuronenpopulation positioniert sein. Vorzugsweise wird nur ein Sensor 3 verwendet, um die Aktivität der zu desynchronisierende Neuronenpopulation zu detektieren. Dadurch wird die Anzahl der zu implantierenden Sensoren möglichst gering gehalten, um unnötigen. Gewebsschädigungen und vor allem einer Hirnblutung während der Implantation vorzubeugen. Es können aber auch beispielsweise zwei oder mehr Sensoren eingesetzt werden, um die neuronale Aktivität der zu desynchronisierenden Neuronenpopulation als Kombination der gemessenen Aktivitäten viel vollständiger zu rekonstruieren.

Des weiteren werden mögliche durch die Implantation verursachte Hirnschädigungen weiter reduziert oder vermieden, und der Stimulationseffekt verbessert, indem Sensoren 3 und Stimulationselektroden 2 in einer zu implantierenden Elektrode kombiniert werden.

### 4.2.1 Ausführungsform für den Fall, dass die Sensoren 3 alle in der zu desynchronisierenden Nervenzellpopulation positioniert sind

Die Sensoren 3 sollen vorzugsweise so angeordnet sein, dass mit den Sensoren ein großer Teil der zu desynchronisierenden Nervenzellpopulation erfasst werden kann. Dies kann mit unterschiedlicher geometrischer Anordnung der Sensoren im Hinblick auf das zu desynchronisierende Gewebe realisiert werden. Im Falle einer Anordnung mit nur einem Sensor 3 kann dieser beispielsweise im Zentrum des Gewebes lokalisiert werden. Im Falle von Anordnungen mit mehreren Sensoren können, wie in Abschnitt 4.1.1 beschrieben, die Sensoren in einer ähnlichen Weise angeordnet werden wie für die Stimulationselektroden beschrieben wurde.

**4.2.2 Ausführungsform für den Fall, dass mindestens einer der Sensoren 3 nicht in der zu desynchronisierenden Nervenzellpopulation positioniert ist** Bei dieser Form der Aktivitätsmessung wird eine der neuronalen Aktivität der zu desynchronisierenden Neuronenpopulation zugeordnete Aktivität in mindestens einem, von dem zu desynchronisierenden Areal, verschiedenen Areal gemessen. Hierbei kann, wie in Abschnitt 3.1 beschrieben, die indirekte Messung durch die Messung der Aktivität einer von der zu desynchronisierenden Nervenzellpopulation verschiedenen Neuronenpopulation und/oder eines Faserbündels und/oder eines Körperteils erfolgen, welches mit der zu desynchronisierenden Nervenzellpopulation verbunden ist.

## 5 Selbstregulierende Bedarfssteuerung der Stimulationsamplitude

Eine der wichtigsten Eigenschaften des Mechanismus der erfindungsgemäßen Vorrichtung ist eine selbstregulierende Bedarfssteuerung der Amplitude des dem zu desynchronisierenden Areal applizierten Stimulationssignals. Die beschriebene Selbstregulierung erfolgt dadurch, dass die applizierten Einzelreize aus der bearbeiteten neuronalen Aktivität bestehen. Im Falle einer stärkeren synchronen Aktivität im zu desynchronisierenden Areal ist, wie dem Fachmann bekannt, eine große Varianz der gemessenen neuronalen Aktivität zu erwarten. Dies führt direkt zu einer erfindungsgemäß zeitverzögerten, Stimulation mit einer erhöhten stimulationsamplitude. Nach Erreichen einer Desynchronisation wird nur noch eine neuronale Aktivität geringer Varianz erwartet, wodurch die Stimulationsamplitude direkt beeinflusst und selbstständig reduziert wird. Tritt wieder eine Resynchronisation ein, so trägt die erfindungsgemäße Vorrichtung dem gesteigerten Bedarf nach desynchronisierender Stimulation automatisch Rechnung, indem die größere Varianz der neuronalen Aktivität zur Bildung stärkerer Einzelreize führt. Dies stellt eine selbstregulierende Bedarfssteuerung der erfindungsgemäßen Vorrichtung dar, siehe auch Abbildung 2c.

Der der selbstregulierenden Bedarfssteuerung zugrunde liegende Mechanismus wirkt in allen im folgenden näher beschriebenen Ausführungsformen der erfindungsgemäßen Vorrichtung.

## 6 Steuerung der Reizapplikation

Unter der zeitlichen Steuerung der Reizapplikation wird eine vorzugsweise im voraus programmierte Ausführungsform der erfindungsgemäßen Vorrichtung verstanden, wobei die Gesamtreize auf eine bestimmte Art mittels der Stimulatoreinheit 8 appliziert werden. Die Varianten der zeitlichen Steuerung der Reizapplikation sind permanente, wiederkehrende und bedarfsgesteuerte Reizapplikation. Zusätzlich kann eine manuelle Bedarfssteuerung, zum Beispiel für eine durch den Patienten oder Arzt durchgeführte Reizapplikation, implementiert werden.

### 6.1 Permanente Reizapplikation

Bei der permanenten Reizapplikation verfügt die erfindungsgemäße Vorrichtung über eine Steuerung, die so programmiert ist, dass sie an den Elektroden 2 eine fortdauernde Applikation der Stimulationssignale vornimmt. Die permanente Reizapplikation stellt die einfachste und leicht zu realisierende Ausführungsform der erfindungsgemäßen Vorrichtung dar. Gleichzeitig erfolgt aufgrund der in Abschnitt 5 beschriebenen erfindungsgemäßen selbstregulierenden Bedarfssteuerung eine gute desynchronisierende Wirkung der permanenten Stimulation bei geringem Energieeintrag in die Zielpopulation. Während der permanenten Reizapplikation kann eine Anpassung der Intensitätsparameter gemäß Abschnitt 7.2.3 stattfinden. Ebenso kann eine Anpassung der Zeitparameter - Stimulationsperiode T und/oder Zeitverzögerungen - während der permanenten Stimulation gemäß Abschnitt 7.2.1 und 7.2.2 in Kombination mit einer Anpassung der Stimulationsintensität oder unabhängig davon erfolgen.

### 6.2 Wiederkehrende Reizapplikation

Bei der wiederkehrenden Reizapplikation verfügt die erfindungsgemäße Vorrichtung über eine Steuerung, die so programmiert ist, dass sie an den Elektroden 2 eine Applikation der Stimulationssignale nur während bestimmter Zeitintervalle vornimmt. Außerhalb dieser Zeitintervalle erfolgt keine Stimulation.

Bei der wiederkehrenden Reizapplikation können die Gesamtreize zeitlich streng periodisch oder zeitlich nicht periodisch verabreicht werden. In dieser Ausführungsform verfügt die erfindungsgemäße Vorrichtung über eine Steuerung, die so programmiert ist, dass sie die Zeitabstände zwischen den Stimulationsintervallen und/oder die Dauer der Intervalle periodisch und/oder nicht periodisch kontrolliert. Eine zeitlich nicht periodische Abfolge der Gesamtreize kann durch einen stochastischen und/oder deterministischen Algorithmus generiert werden, um den gewünschten desynchronisierten Zustand der zu desynchronisierenden Population zu erreichen. Die Stimulations- und Messintervalle können überlappend oder zeitgleich oder zeitlich getrennt angeordnet sein.

Während der wiederkehrenden Reizapplikation kann eine Anpassung der Intensitätsparameter gemäß Abschnitt 7.2.3 stattfinden. Ebenso kann eine Anpassung der Zeitparameter - Stimulationsperiode T und/oder Zeitverzögerungen - während der wiederkehrenden Stimulation gemäß Abschnitt 7.2.1 und 7.2.2 in Kombination mit einer Anpassung der Stimulationsintensität oder unabhängig davon erfolgen.

### 6.3 Bedarfsgesteuerte Reizapplikation,

Bei der bedarfsgesteuerten Reizapplikation verfügt die erfindungsgemäße Vorrichtung über eine Steuerung, die so programmiert ist, dass sie das An- und Abschalten der Stimulationssignale entsprechend der bestimmten Zustände der zu desynchronisierenden Neuronenpopulation vornimmt. Das Anschalten der Stimulation erfolgt beispielsweise wie im Folgenden beschrieben.

Über Sensor 3 wird die Aktivität der zu desynchronisierenden Neuronenpopulation gemessen. Die neuronale Aktivität wird an die Einheit 4 zur Signalverarbeitung und/oder Regelung weitergeleitet, die unter anderem als Mittel zum Erkennen eines pathologischen Merkmals fungiert. Sobald die Einheit 4 zur Signalverarbeitung und/oder Regelung in der neuronalen Aktivität ein pathologisches Merkmal erkennt, wird die Applikation eines Gesamtreizes gestartet. Vorzugsweise wird, sobald das pathologische Merkmal aufgrund der Wirkung der applizierten Stimulation verschwindet, die Stimulation abgeschaltet. Die erfindungsgemäße Vorrichtung umfasst daher in einer möglichen Ausführungsform als Einheit 4 zur Signalverarbeitung und/oder Steuerung/Regelung einen Rechner, welcher einen Datenträger beinhaltet, der die Daten des Krankheitsbildes trägt und mit den Messdaten vergleicht. Unter den Daten des Krankheitsbildes werden für die Stimulation relevante Parameter und Messgrößen verstanden, zum Beispiel die Momentanfrequenz der über Sensor 3 gemessenen neuronale Aktivität, des für die Verfahrensweise der bedarfsgesteuerten Reizapplikation notwendigen Schwellenwerts, die Stimulationsparameter, welche die Reizstärke festlegen. Unter einem pathologischen Merkmal ist beispielsweise eine krankheitsbedingte Synchronisation der zu desynchronisierenden Neuronenpopulation zu verstehen und kann durch folgende Eigenschaften der neuronalen Aktivität erkannt werden:

a) Falls über den Sensor 3 ausschließlich bzw. überwiegend die pathologische Aktivität der zu desynchronisierenden Neuronenpopulation und/oder einer mit dieser Neuronenepopulation eng verbundenen Neuronenpopulation und/oder eng verbundenem Teil des Nervensystems oder der Muskulatur gemessen wird, wie z.B. bei der in Abschnitt 3.1 und Abschnitt 4.2.1 beschriebenen direkten Messung, wird die neuronale Aktivität direkt zur Feststellung, ob die Amplitude der neuronalen Aktivität einen Schwellenwert überschreitet, verwendet. Die erfindungsgemäße Vorrichtung ist daher in einer bevorzugten Ausführungsform mit Mitteln zum Erkennen eines dem Schwellenwert entsprechenden Wertes der Amplitude der neuronalen Aktivität ausgestattet. In diesem Fall wird vorzugsweise die neuronale Aktivität selbst und/oder ihr Betrag und/oder ihre Amplitude mit dem Schwellenwert verglichen. Das Mittel zum Erkennen des Schwellenwertes kann bei dieser Ausführungsform so programmiert sein, dass es beispielsweise die neuronale Aktivität selbst und/oder ihr Betrag und/oder ihre Amplitude mit dem Schwellenwert vergleicht. Die Bestimmung der Amplitude erfolgt entweder in einer einfachen Version mittels Bestimmung des Betrags des Signals, und/oder mit Bandpassfilterung und nachfolgender Hilbert-Transformation oder Wavelet-Analyse. Die Einheit 4 zur Signalverarbeitung und/oder Regelung ist in diesem Fall so programmiert, dass sie eine Bestimmung des Betrags des Signals und/oder eine Bandpassfilterung mit Hilberttransformation und/oder eine Wavelet-Analyse durchführen kann. Die neuronale Aktivität oder ihr Betrag wird besonders bevorzugt verwendet, da die Berechnung der Amplitude einen deutlich höheren Rechenaufwand bedeutet, und die Bestimmung der Amplitude nicht auf einem einzelnen Messwert der neuronalen Aktivität durchgeführt werden kann, sondern in einem hinreichend großem, dem Fachmann bekannten Zeitintervall durchgeführt werden muss, was die Erkennung des pathologischen Merkmals etwas verzögern kann.

b) Falls über Sensor 3 neben dieser pathologischen Aktivität der zu desynchronisierenden Neuronenpopulation zusätzlich noch nicht krankheitsspezifische Aktivität, zum Beispiel aus anderen Neuronenpopulationen, gemessen wird, wie z.B. bei der in den Abschnitten 3.1 und 4.2.2 beschriebenen indirekten Messung, muss bei der Analyse der neuronalen Aktivität ein weiterer algorithmischer Schritt eingefügt werden. Da die krankheitsspezifische Aktivität typischerweise in einem Frequenzbereich auftritt, der von Frequenzbereich der nicht krankheitsspezifischen Aktivität verschieden ist, genügt es hierzu vorzugsweise eine Abschätzung der Aktivität im krankheitsspezifischen Frequenzbereich durchzuführen. Die Frequenz der krankheitsspezifischen Aktivität wird beispielsweise durch eine Bestimmung der Differenz von aufeinander folgenden Triggerpunkten durchgeführt. Triggerpunkte sind Punkte, wie Maxima, Minima, Wendepunkte und Nulldurchgänge. Vorzugsweise wird diese Analyse in einem gleitenden Zeitfenster durchgeführt, wobei der Mittelwert von mehreren zeitlichen Differenzen gebildet wird, wodurch die Stabilität erhöht wird. Alternativ kann die Frequenzschätzung auch mit den dem Fachmann bekannten spektralen Schätzmethoden und anderen Frequenzschätzern bestimmt werden. Hierzu verfügt die erfindungsgemäße Vorrichtung in einer besonderen Ausführungsform Mittel zur Abschätzung der Aktivität im krankheitsspezifischen Frequenzbereich, wie spektrale Schätzmethoden, Wavelet-Analyse u.s.w.. Dies wird beispielsweise durch Mittel zum Durchführen einer Frequenzanalyse realisiert. Es kann beispielsweise die spektrale Energie im krankheitsspezifischen Frequenzbereich in einem gleitenden Fenster bestimmt werden. Alternativ kann nach Bandpassfilterung die Amplitude im krankheitsspezifischen Frequenzbereich durch Bestimmung des Maximums des bandpassgefilterten Signals oder durch Bestimmung des Mittelwerts des Betrags des bandpassgefilterten Signals oder mit nachfolgender Hilbert-Transformation oder mittels Wavelet-Analyse ermittelt werden. Hierzu umfasst die erfindungsgemäße Vorrichtung beispielsweise Mittel zur Bandpassfilterung der Amplitude und Mittel zur Bestimmung des Maximums des bandpassgefilterten Signals und/oder Mittel zur Bestimmung des Mittelwerts des Betrags des bandpassgefilterten Signals und/oder Mittel zur Durchführung einer Hilberttransformation und/oder einer Wavelet-Analyse.

Bei bedarfsgesteuerter Reizapplikation wird beispielsweise immer derselbe Gesamtreiz verwendet. Vorzugsweise wird die Stimulationsperiode T wie in Abschnitt 7.2.1 beschrieben an die momentane Frequenz der zu desynchronisierenden Neuronenpopulation angepasst. Es wird dann bei Vorliegen des pathologischen Merkmals ein Reiz mit an die momentane Frequenz angepasster Stimulationsperiode T appliziert. Ebenso können die Zeitverzögerungen gemäß Abschnitt 7.2.2 angepasst werden und/oder die Intensität dieses Reizes bleibt dabei vorzugsweise konstant. Die Intensitätsparameter können aber auch, wie in Abschnitt 7.2.3, gemäß des Stimulationseffekts modifiziert werden.

### 6.3.1 Feststellung des Bedarfs

Aus mindestens zwei Gründen gibt es keine eineindeutige Beziehung zwischen der Ausprägung des pathologischen Merkmals und der Ausprägung der krankheitsspezifischen Symptome. Zum einen bedingt die Entfernung von Sensor 3 zu dem zu desynchronisierenden Areal, in welchem die zu messende neuronale Aktivität generiert wird, eine Veränderung der Amplitude im krankheitsspezifischen Frequenzbereich. Zum anderen ist eine bestimmte Ausprägung des krankheitsspezifischen Merkmals, das heißt die Ausprägung der rhythmischen Aktivität im krankheitsspezifischen Frequenzbereich, nicht eineindeutig mit den krankheitsspezifischen Symptomen verbunden. Da der krankheitsspezifische Rhythmus Auswirkungen auf komplexe Nervennetzwerke im Gehirn hat, die typischerweise obendrein nicht einfachen linearen dynamischen Gesetzmäßigkeiten gehorchen, gelten keine eineindeutigen Relationen zwischen krankheitsspezifischem Rhythmus und Ausprägung der Symptome. Wenn zum Beispiel der krankheitsspezifische Rhythmus nicht hinreichend mit der biomechanisch vorgegebenen Eigenfrequenz einer Extremität übereinstimmt, ist der durch den krankheitsspezifischen Rhythmus bedingte Tremor deutlich geringer, als wenn der krankheitsspezifische Rhythmus in Resonanz mit der biomechanisch vorgegebenen Eigenfrequenz der Extremität übereinstimmt.

Die charakteristischen Eigenschaften, wie z. B.

die dominierende Frequenz und die Amplitude, der gemessenen neuronalen Aktivität befinden sich in einem dem Fachmann bekannten Erfahrungsbereich. Der Wert der Ausprägung des krankheitsspezifischen Merkmals der über Sensor 3 gemessenen neuronalen Aktivität wird als Schwelle bezeichnet, bei dessen Überschreiten es typischerweise zum Auftreten von Symptomen, zum Beispiel des Tremors, kommt. Die Schwelle ist ein Parameter, der für die Ausführungsform der in Abschnitt 6.3 beschriebenen bedarfsgesteuerten Reizapplikation gewählt werden muss. Die erfindungsgemäße Vorrichtung umfasst daher in Form der Steuereinheit 4 Mittel zum Erkennen eines Schwellenwertes. Mit dem erfindungsgemäßen Verfahren der bedarfsgesteuerten Reizapplikation wird der Vorteil erreicht, dass die Wirksamkeit der erfindungsgemäßen Vorrichtung nicht kritisch von der Wahl der Schwelle abhängt, sondern bezüglich der Wahl der Schwelle eine große Fehlertoleranz gegeben ist, die beispielsweise in einem Bereich von bis zu 50 % der maximalen Ausprägung des krankheitsspezifischen Merkmals liegt. Die Wahl der Schwelle wird entweder intraoperativ oder vorzugsweise in den ersten Tagen nach der Operation durch Messung der neuronalen Aktivität über Sensor 3, mit Bestimmung der Ausprägung des krankheitsspezifischen Merkmals und Vergleich mit der Ausprägung der Symptome, z. B. der Stärke des Zitterns, bestimmt.

In einer weniger bevorzugten Ausführungsform der bedarfsgesteuerten Reizapplikation wird als Schwelle ein repräsentativer Wert, zum Beispiel der Mittelwert, eines Kollektivs von bei Patienten gemessenen Schwellenwerten genommen.

In einer bevorzugten Ausführungsform wird die Wahl der Schwelle in im wesentlichen regelmäßigen Abständen, zum Beispiel im Rahmen von halbjährlichen Kontrollen, überprüft .

In der in Abschnitt 6.2 beschriebenen Ausführungsform der wiederkehrenden Stimulation mit bedarfsgesteuerter Reizstärke ist keine Schwellenwertdetektion notwendig.

Die oben beschriebenen drei Stimulationsmethoden können vorzugsweise in unterschiedlicher Kombination mit den in Abschnitt 7.2 beschriebenen Methoden zur Anpassung der Stimulationsparameter verwendet werden.

Allen drei Stimulationsmethoden gemeinsam ist die inhärente erfindungsgemäße selbstregulierende Bedarfssteuerung. Die direkte Abhängigkeit der Stimulationssignale von der gemessenen neuronalen Aktivität bedingt eine, in Abschnitt 5 beschriebene, selbstregulierende Bedarfssteuerung, wodurch der Energieeintrag in die Zielpopulation minimiert wird. Diese selbstregulierende Bedarfssteuerung wirkt unabhängig von der Realisation der in Abschnitt 6.3 beschriebenen zusätzlichen Bedarfssteuerung und der Kalibrierung und Regelung der Parameter, wie sie in Abschnitt 7 beschrieben wird.

## 7 <u>Kalibrierung und Anpassung der Parameter</u>

Im Folgenden wird davon ausgegangen, dass alle Elektroden 2 in der zu desynchronisierenden Neuronenpopulation liegen. Der Fall, dass mindestens eine Elektrode außerhalb der zu desynchronisierenden Neuronenpopulation liegt wird gesondert am Ende des Abschnitts betrachtet. Für folgende Parameter der erfindungsgemäßen Vorrichtung kann beispielsweise eine Kalibrierung und Anpassung durchgeführt werden: die Frequenz des Stimulationssignals, deren Kehrwert der Stimulationsperiode entspricht, die Zeitverzögerungen der Einzelreize und die Intensität der Einzelreize.

### 7.1 Stimulationsparameter zu Beginn der Stimulation

#### 7.1.1 Frequenz, Stimulationsperiode

Wahl der Frequenz ohne vorherigen Betrieb der Vorrichtung: Der Frequenzbereich der pathologischen neuronalen Aktivität ist für die jeweiligen Krankheitsbilder dem Fachmann bekannt (Elble R.J. und Koller W.C. (1990): Tremor, John Hopkins University Press, Baltimore). Von diesem Frequenzbereich kann vorzugsweise der Mittelwert genommen werden. Alternativ kann stattdessen aus einer Datenbank der alters- und geschlechtsspezifisch zu erwartende Wert der Frequenz verwendet werden.

Es ist für den erfolgreichen Betrieb der erfindungsgemäßen Vorrichtung nicht notwendig, dass die anfänglich vorgegebene Frequenz mit der tatsächlich vorhandenen Frequenz der Aktivität der zu desynchronisierenden Neuronenpopulation übereinstimmt. Die unter 7.2.1 beschriebene Regelung der Stimulationsperiode T funktioniert auch, wenn ein vom richtigen Frequenzwert stark abweichender Anfangswert verwendet wird. Hierbei bedeutet stark abweichend, dass der Wert auch um mindestens einen Faktor 10 zu groß bzw. zu klein sein kann. Alternativ kann somit auch vorzugsweise mit einem Frequenzwert begonnen werden, der in dem, dem Fachmann bekannten, für die Krankheit typischen Frequenzbereich liegt. Der Wert der Frequenz zu Beginn der Stimulation kann auch vorzugsweise durch eine individuelle Anpassung an den jeweiligen Patienten gewonnen werden. Dies kann beispielsweise durch eine die Stimulation vorbereitende Messung der neuronalen Aktivität und Abschätzung der dominierenden Frequenz der Aktivität der zu desynchronisierenden Neuronenpopulation, wie in Abschnitt 6.3b beschrieben, realisiert werden.

Wahl der Frequenz mit vorherigem Betrieb der Vorrichtung: Als Startwert für die Frequenz wird der Mittelwert der Frequenz während des vorhergehenden Betriebs der Vorrichtung gewählt.

In beiden Fällen, das heißt mit und ohne vorherigen Betrieb der Vorrichtung, wird die Stimulationsperiode T berechnet als Kehrwert des Startwerts der Frequenz.

### 7.1.2 Zeitverzögerungen

Die Zeitverzögerungen der Einzelreize werden vorzugsweise nach einer ersten Festlegung der Stimulationsfrequenz bzw. der Stimulationsperiode T bestimmt. Vorzugsweise werden die Zeitverzögerungen als Bruchteile der Stimulationsperiode T gewählt, wobei vorzugsweise jedem Einzelreiz eine unterschiedliche Zeitverzögerung zugewiesen wird. Vorzugsweise werden die Zeitverzögerungen in einer Art bestimmt, so dass die Differenz der Zeitverzögerungen Bruchteilen der Stimulationsperiode T entsprechen, im Falle äquidistanter Zeitverzögerungen würde die Differenz der Zeitverzögerungen somit ein vielfaches von T/N betragen. Äquidistante Zeitverzögerungen sind aber für eine erfolgreiche Desynchronisation der zu desynchronisierenden Neuronenpopulation nicht notwendig. Vorzugsweise können auch Zeitverzögerungen gewählt werden, die einem Vielfachen von Bruchteilen der Stimulationsperiode T entsprechen und die Stimulationsperiode T ggf. überschreiten. Die in Abschnitt 7.2.2 beschriebene Anpassung der Zeitverzögerungen funktioniert auch in dem oben beschriebenen Fall, dass die Zeitverzögerungen die Stimulationsperiode T zumindest teilweise überschreiten.

### 7.1.3 Intensität

Die Ausgangswerte der Stimulationsparameter, welche die Intensität der Einzelreize bestimmen (z. B. Verstärkung des Feedback-Stimulationssignals) werden gemäß den dem Fachmann bekannten Erfahrungswerten (z. B. maximale Amplitude 5 V) festgelegt. Die unter 7.2.3 beschriebene Regelung der Intensität funktioniert auch, wenn ein vom günstigsten Intensitätswert stark abweichender Anfangswert verwendet wird. Hierbei bedeutet stark abweichend, dass der Wert auch um mindestens einen Faktor 10 zu groß (maximale Amplitude 5 V) bzw. zu klein sein kann. Alternativ kann somit auch vorzugsweise mit einem Intensitätswert begonnen werden, der in dem, dem Fachmann bekannten, Bereich liegt. Insbesondere ist es bevorzugt, eine Stimulation mit kleinen Werten der Intensität, beispielsweise Maximalamplitude von 0.5 V, der Stimulationssignale zu beginnen, um somit die Nebenwirkungen der Stimulation möglicherweise zu reduzieren. Falls eine Notwenigkeit, stärkere Stimulationssignale zu benutzen, besteht, kann eine Vergrößerung der Intensität in kleinen Schritten, wie in Abschnitt 7.2.3 beschrieben, durchgeführt werden.

Somit können die Anfangswerte für Frequenz und Intensität vorgegeben werden und müssen, insbesondere nicht im Rahmen einer zeitaufwendigen Kalibrierung, bestimmt werden.

## 7.2 Anpassung der Stimulationsparameter

### 7.2.1 Anpassung der Stimulationsperiode *T*

Im zu desynchronisierenden Areal oder einem damit eng verbundenen Areal wird die neuronale Aktivität gemessen, welche nach einer Bearbeitung als Stimulationssignal verwendet wird. Zum Beispiel kann beim Morbus Parkinson statt einer Messung über die Sensoren 3 direkt im zu desynchronisierenden Areal auch eine Messung der Aktivität in einem nachgeschalteten Areal, z.B. dem prämotorischen Cortex über epikortikale Sensoren erfolgen. In einem Zeitfenster mit unten angegebener Länge wird die dominante mittlere Periode bestimmt. Hierzu können unterschiedliche Algorithmen verwendet werden. Beispielsweise kann die momentane Periode als die zeitliche Differenz zweier nachfolgender Maxima der gemessenen neuronalen Aktivität bestimmt werden. Es kann auch beispielsweise zuerst die mittlere Frequenz der neuronalen Aktivität abgeschätzt werden, und die Stimulationsperiode T als Kehrwert der mittleren Frequenz festgelegt werden. Falls über Sensor 3 nicht nur krankheitsspezifische Aktivität gemessen wird, muss für diese Art der Frequenzschätzung zuerst die krankheitsspezifische Aktivität über eine Bandpassfilterung des für die Krankheit spezifischen Frequenzbereichs extrahiert werden. Alternativ kann beispielsweise die Frequenz über die in Abschnitt 6.3 genannten Frequenzschätzer bestimmt werden. Das für diese Frequenzschätzung verwendete Zeitfenster hat eine Länge, die nach oberen Werten offen sein kann und beispielsweise 10000 Perioden, vorzugsweise 1000 Perioden besonders bevorzugt 100 Perioden der krankhaften Aktivität aber auch anderen beliebigen Werten entspricht.

### 7.2.2 Anpassung der Zeitverzögerungen

Wie in den Abschnitten 3.3, 3.4 und 7.1.2 beschrieben, werden die Zeitverzögerungen der Einzelreize typischerweise als Bruchteile der Stimulationsperiode T gewählt. Während der Stimulation können die Zeitverzögerungen beispielsweise fixiert sein, oder vorzugsweise an die gemäß Abschnitt 7.2.1 angepasste Stimulationsperiode angepasst werden. Um eine optimale Desynchronisation mit geringer Stimulationsintensität erreichen zu können, werden die Zeitverzögerungen der Einzelreize vorzugsweise während der Stimulation durch einen deterministischen oder stochastischen Algorithmus variiert. Hierzu umfasst die erfindungsgemäße Vorrichtung Mittel in Form der Steuereinheit 4, die es erlauben, die Zeitverzögerungen der Einzelreize während der Stimulation zu variieren. Des Weiteren können die Zeitverzögerungen beispielsweise nicht nur innerhalb einer Stimulationsperiode sondern auch im Rahmen mehrerer Perioden variiert werden. In diesem Fall entsprechen die Einzelreize der bearbeiteten neuronalen Aktivität welche zu einem einige Perioden früheren Zeitpunkt gemessen wurde.

### 7.2.3 Anpassung der Intensität

Über Sensor 3 wird die neuronale Aktivität gemessen, welches die Aktivität der zu desynchronisierenden Neuronenpopulation darstellt. Diese neuronale Aktivität wird an die Einheit 4 zur Signalverarbeitung und/ oder Regelung weitergeleitet. Die Einheit 4 zur Signalverarbeitung und/oder Regelung führt eine gemäß Abschnitt 6 permanente oder wiederkehrende oder bedarfsgesteuerte Stimulation durch, wobei die Stärke der zum jeweiligen Zeitpunkt applizierten Gesamtreize von der Ausprägung des pathologischen Merkmals in der neuronalen-Aktivität abhängt. Zu diesem Zweck kann die Intensität vorzugsweise angepasst werden. Die Relation zwischen Reizstärke und Ausprägung des pathologischen Merkmals kann entweder manuell oder in Abhängigkeit vom Stimulationserfolg automatisch geregelt werden. In einem Zeitfenster frei wählbarer, vorzugsweise konstanter Länge, das in einem konstanten Zeitabstand vor dem jeweiligen Reiz endet, wird die Ausprägung des pathologischen Merkmals in folgender Weise ermittelt:

a) In dem Fall, wenn über Sensor 3 ausschließlich bzw. überwiegend die zu desynchronisierende pathologische Aktivität und/oder eine damit eng verbundene neuronale bzw. muskuläre Aktivität gemessen wird, entspricht die Amplitude der Ausprägung der Synchronisation der zu desynchronisierenden Neuronenpopulation. Die Amplitude repräsentiert somit das pathologische Merkmal. Die Amplitude kann dabei abgeschätzt werden über die Bestimmung des Maximums des Signals oder über den Mittelwert des Betrags des Signals oder mit Bandpassfilterung mit nachfolgender Hilbert-Transformation oder Wavelet-Analyse. Die ersten beiden Varianten (Bestimmung des Maximums des Signals oder Bestimmung des Mittelwerts des Betrags des Signals) werden besonders bevorzugt verwendet, da die Berechnung der Amplitude mittels Hilbert-Transformation oder Wavalet-Analyse einen deutlich höheren Rechenaufwand bedeutet und deren Genauigkeit von der richtigen Auswahl algorithmischer Parameter abhängt.

b) Falls über Sensor 3 neben der krankheitsspezifischen Aktivität zusätzlich noch nicht krankheitsspezifische Aktivität, zum Beispiel aus anderen Neuronenpopulationen, gemessen wird, kann für die Abschätzung der Ausprägung des pathologischen Merkmals die neuronale Aktivität nicht direkt angewandt werden. Da die krankheitsspezifische Aktivität typischerweise in einem Frequenzbereich auftritt, der von dem Frequenzbereich der nichtkrankheitsspezifischen Aktivität verschieden ist, wird in diesem Falle vorzugsweise eine Abschätzung der Aktivität im krankheitsspezifischen Frequenzbereich durchgeführt. Dies wird beispielsweise durch eine Frequenzanalyse realisiert. Es kann beispielsweise die spektrale Energie im krankheitsspezifischen Frequenzbereich bestimmt werden. Alternativ hierzu kann nach Bandpassfilterung die Amplitude durch die Bestimmung des Maximums des bandpassgefilterten Signals oder durch die Bestimmung des Mittelwerts des Betrags des Signals oder mit nachfolgender Hilbert-Transformation oder mit Wavelet-Analyse bestimmt werden.

Wird der gewünschter Effekt nicht erzielt, das heißt, wird die Zielpopulation nicht in ausreichendem Maße desynchronisiert und somit der pathologische Merkmal der neuronalen Aktivität nicht unter den Schwellenwert verschoben, wird die maximale Stärke des Stimulus bis zu einem aus Sicherheitsgründen starr vorgegebenen Maximalwert, zum Beispiel 5V, langsam erhöht (z. B. in Schritten von 0,5 V pro 50 Perioden). Hierzu verfügt die erfindungsgemäße Vorrichtung über eine Steuerung, welche eine Änderung der neuronalen Aktivität erkennt und beim Ausbleiben der Änderung der neuronalen Aktivität die stimulierenden Signale nach oberen Werten anpasst. Nach ca. 20 erfolgreichen Perioden der Stimulation kann die Vorrichtung beginnen, die maximale Stärke des Stimulus langsam (z. B. in Schritten von 0,5 V pro 50 Perioden) so lange nach unten regeln, solange der Stimulationserfolg noch vorhanden ist. Dabei wird der Stimulationserfolg wie oben beschrieben ermittelt. Die Steuerung ist dabei so programmiert, dass sie die Änderung der neuronalen Aktivität und somit den Stimulationserfolgt erkennt. Vorzugsweise wird die maximale Reizstärke auf einer Zeitskala zwischen 10 und 1000 Perioden der neuronalen Aktivität so geregelt, dass die zu desynchronisierende Neuronenpopulation ausreichend desynchronisiert wird.

Unabhängig von dem Wert der oben definierten Stimulationsintensität wird die Amplitude des Stimulationssignals infolge der in Abschnitt 5 beschriebenen Eigenschaften des Stimulationsmechanismus der erfindungsgemäßen Vorrichtung nach erfolgreicher Desynchronisation selbständig minimiert.

### 7.3 Stimulationsparameter für den Fall, dass mindestens eine Elektrode 2 nicht in der zu desynchronisierenden Neuronenpopulation liegt

Wie im in Abschnitt 3.3 beschriebenen Fall einer nicht in der zu desynchronisierenden Neuronenpopulation gelegenen Elektrode 2, wird die zu desynchronisierende Neuronenpopulation über eine indirekte Stimulation beeinflusst, wie in Abschnitt 4.1.2 beschrieben. Da im Falle einer indirekten Stimulation die Leitungszeiten zwischen den stimulierten Neuronenpopulationen einerseits und der zu desynchronisierenden Neuronenpopulation andererseits jeweils verschieden groß sein können, werden vor der Durchführung der desynchronisierenden Stimulation zuerst die jeweiligen Leitungszeiten gemessen. Hierzu wird über jeweils eine Stimulationselektrode 2 gereizt und die Reizantwort über die, in der zu desynchronisierenden Neuronenpopulation platzierten Sensoren 3, gemessen. Dies wird bei allen Stimulationselektroden 2, über die indirekt stimuliert wird, separat n mal durchgeführt, wobei n typischerweise eine kleine ganze Zahl bis zu beispielsweise 200 ist. Hieraus wird die mittlere Leitungszeit vorzugsweise in folgender Weise abgeschätzt:

Die Dauer zwischen Beginn der Stimulusapplikation über die j-te Elektrode 2 und dem ersten Maximum der Reizantwort bzw. des Betrags der Reizantwort, $\tau_j^{(k)}$, wird für jede einzelne Reizapplikation bestimmt. Bei $\tau_j^{(k)}$ steht der Index j für die j-te Elektrode 2, während der Index k für den k-ten applizierten Stimulus steht.

Hieraus wird dann für jede Stimulationselektrode 2, über die indirekt stimuliert wird, separat die mittlere Dauer zwischen Reizbeginn und Reizantwort nach folgender Formel 1 bestimmt:

$$\bar{\tau}_j = \frac{1}{L_j} \sum_{k=1}^{L_j} \tau_j^{(k)} .$$

Formel 1

Hierbei ist $L_j$ die Anzahl der über die j-te Stimulationselektrode 2 applizierten Reize. $L_j$ kann, aber muss nicht für alle Stimulationselektroden 2, über die indirekt stimuliert wird, gleich sein.

Für die Stimulation wird die auf diese Weise bestimmte Leitungszeit $\bar{\tau}_j$ in folgender Weise berücksichtigt: Würde bei direkter Stimulation der zu desynchronisierenden Neuronenpopulation mit einer Zeitverzögerung $t$ über die j-te Stimulationselektrode 2 ein Reiz appliziert, so wird bei indirekter Stimulation über die j-te Stimulationselektrode 2 der Reiz mit einer Zeitverzögerung $t-\bar{\tau}_j$ verabreicht, wobei t größer als $\bar{\tau}_j$ sein muss, was gemäß Abschnitt 7.2.2 realisiert werden kann.

Die Bestimmung der Stimulationsparameter zu Beginn der Stimulation und die Regelmechanismen während der Stimulation werden unter der oben beschriebenen Berücksichtigung der Leitungszeiten $\bar{\tau}_j$ völlig analog, wie in den Abschnitten 7.1 und 7.2 beschrieben, durchgeführt.

## 8 Vorteile

Die erfindungsgemäße Vorrichtung hat mehrere Vorteile im Vergleich mit existierenden Vorrichtungen, z.B. DE 103 18 071.0-33 "Vorrichtung zur Desynchronisation von neuronaler Hirnaktivität":

1. Der Hauptvorteil der erfindungsgemäßen Vorrichtung besteht darin, dass ein physiologischer Reiz, nämlich das Feedback-Stimulationssignal, das heisst, die gemessene und bearbeitete neuronale Aktivität der zu desynchronisierenden Neuronenpopulation, für die Stimulation benutzt wird. Dadurch findet die in Abschnitt 5 beschriebene selbstregulierende Bedarfssteuerung der Stimulationsamplitude statt, was den Energieeintrag in die zu desynchronisierende Neuronenpopulation minimiert und zu geringen Nebenwirkungen führt.

2. Aufgrund der selbstregulierenden Stimulationsamplitude gemäß Abschnitt 5 ist der Betrieb der erfindungsgemäßen Vorrichtung energiesparend, da sowohl, aufgrund der bedarfsgesteuerten Stimulationsamplitude, ein energiesparendes Signal zur Stimulation verwendet wird, als auch eine Energieeinsparung bei den für die Stimulationssteuerung notwendigen erfindungsgemäßen Steuervorrichtungen erwartet werden kann. Dadurch können längere Intervalle zwischen den notwendigen und für den Patienten beschwerlichen Batteriewechseln realisiert werden.

3. Besonders vorteilhaft ist die Ausführungsform der wiederkehrenden oder permanenten Applikation mit bedarfsgesteuerter Reizstärke, da bei diesem Verfahren keine Schwelle detektiert werden muss. Dadurch ist diese Ausführungsform mit deutlich einfacheren Algorithmen realisierbar. Dementsprechend ist ihre software- bzw. hardwaremäßige Realisation deutlich weniger aufwändig.

4. Bei permanenter und wiederkehrender Stimulation mit bedarfsgesteuerter Reizstärke und direkter Stimulation der zu desynchronisierenden Neuronenpopulation ist keine Kalibrierung nötig, d.h. es muss keine Serie von Testreizen durchgeführt werden, bei der Stimulationsparameter systematisch variiert werden, was zu einer verringerten Dauer der Kalibrierung führt.

5. Die erfindungsgemäß durchgeführte Kalibrierung ist schneller, weniger störanfällig und weniger aufwändig, da bei direkter Stimulation ohne Testreizung mit dem Stimulationsbetrieb begonnen werden kann, wobei im Laufe des Stimulationsbetriebs die Parameter wie in Abschnitt 7.2 beschrieben optimiert werden. Auf Grund der schnell durchführbaren Kalibrierung kann die erfindungsgemäße Vorrichtung schon intraoperativ angewandt werden, wodurch die Platzierung der Tiefenelektroden 2 optimiert wird. Es ist auf diese Weise möglich, die Auswirkung der desynchronisierenden Stimulation auf die Ausprägung der Symptome, zum Beispiel den Tremor, direkt als Parameter, für die Güte der Platzierung, zu verwenden.

6. Weniger störanfällig ist die erfindungsgemäße Kalibrierung, da die im Rahmen der erfindungsgemäßen Kalibrierung verwendeten Frequenz- und Leitungszeitschätzer nicht kritisch von Parametern, wie zum Beispiel den Grenzen und der Charakteristik eines Bandpassfilters, abhängen. Dadurch ist diese Ausführungsform mit deutlich einfacheren Algorithmen realisierbar. Dementsprechend ist ihre software- bzw. hardwaremäßige Realisation deutlich weniger aufwändig.

7. Insgesamt von großem Vorteil ist die allgemeine Toleranz und Robustheit der erfindungsgemäßen Vorrichtung gegenüber der Abschätzung der Parameter Intensität, Stimulationsperiode und Zeitverzögerungen.

8. Unter Ausnutzung der möglicherweise unterschiedlichen Stimulationswirkung in Abhängigkeit von der Entfernung zwischen Elektrode und zu stimulierendem Areal und unter Ausnutzung der pathologisch gesteigerten Kopplung zwischen den Neuronen der zu desynchronisierenden Neuronenpopulation, stabilisiert die erfindungsgemäße Vorrichtung das zu desynchronisierende Areal in einen desynchronisierten und angestrebten Zustand. Dieser Zustand wird dauerhaft realisiert und bringt das zu desynchronisierende Areal dadurch sehr nahe an den physiologischen Zustand. Beispielsweise ergibt sich eine anhaltende Desynchronisation ohne Hin- und Herschwanken zwischen Resynchronisation und Clusterzustand.

Beispiel:

Wird zum Beispiel an vier Orten stimuliert, so können über die vier Elektroden beispielhaft folgende Reize abgegeben werden:

1. Über jede der Elektroden wird das Feedback-Stimulationssignal, d.h. die bearbeitete neuronale Aktivität, appliziert, wobei, wie in Figur 3 gezeigt die Stimulationssignale jeweils zeitlich um T/4 versetzt sind, wobei T die mittlere Periode des Rhythmus der zu desynchronisierenden Neuronenpopulation ist.

2. Über die Elektroden 1 und 2 werden, wie in Figur 4 dargestellt, Stimulationssignale gleicher Zeitverzögerung aber unterschiedlicher Polarität appliziert. Ebenso werden über die Elektroden 3 und 4 dieselben Stimulationssignale unterschiedlicher Polarität appliziert.

Es wird beispielhaft mit drei unterschiedlichen, in Abschnitt 6 beschriebenen, Kontrollmechanismen der Reizapplikation stimuliert, mit denen vorzugsweise, wie in Abschnitt 7 beschrieben, eine bedarfsgesteuerte und somit energiesparende und milde (Nebenwirkungen vermeidende) Stimulation ermöglicht wird:

1. Permanente Reizapplikation: Es wird permanent stimuliert, siehe Figur 2, vorzugsweise mit Anpassung der Stimulationsperiode. Wie in Figur 2 beispielhaft zu sehen, tritt direkt nach Applikation der Stimulation eine Desynchronisation der zu desynchronisierenden Neuronenpopulation auf. Dadurch wird die Amplitude der gemessenen neuronalen Aktivität, siehe Figur 2b, minimiert. Gleichzeitig tritt, wie in Figur 2c beispielhaft zu sehen, eine Minimierung der Stimulationsamplitude aufgrund des in Abschnitt 5 beschriebenen Mechanismus der selbstregulierenden Bedarfssteuerung ein. Nach Ausschalten der Stimulation tritt aufgrund der pathologischen Interaktion zwischen den Neuronen der Population nach kurzer Zeit eine Resynchronisation ein.

2. Bedarfsgesteuerte Reizapplikation (d.h. bedarfsgesteuerte Wahl der Start- und Endzeitpunkts der Stimulation) der Gesamtreize: Wenn die Synchronisation der Nervenzellpopulation einen Schwellenwert überschreitet, wird der nächste Gesamtreiz über alle Elektroden abgegeben, wie in Abschnitt 6.3 beschrieben.

3. Wiederkehrende Reizapplikation: Es erfolgt eine wiederkehrende Stimulation mit koordinierten Reizen über alle Elektroden. Dabei wird die Stärke der Reize an die Stärke der Synchronisation der Neuronenpopulation angepasst: Je stärker die Synchronisation, desto stärker ist der koordinierte Reiz.
Bei dieser Variante kann man als Zeitverzögerung zwischen den Einzelreizen statt T/4 vorzugsweise $\tau/4$ wählen, wobei $T$ die Periode des Rhythmus ohne Stimulation und $\tau$ die durch Stimulation dem Rhythmus aufgezwungene Periode ist. Mit anderen Worten: $1/\tau$ ist die Frequenz des Stimulationssignals, mit der die Einzelreize appliziert werden. Hierdurch zwingt man dem System den einzigen kritischen Stimulationsparameter auf: Anstatt diesen im Rahmen einer aufwendigen Kalibrierung geeignet zu bestimmen, wird er durch die Stimulation diktiert. Außerdem wird bei dieser Form der bedarfsgesteuerten Stimulation der Umstand ausgenützt, dass die Neuronen in den betroffenen Gebieten eine (krankhafte) Tendenz zu periodischem Feuern bzw. Bursten (rhythmische Produktion von Gruppen von Aktionspotentialen) haben. Deswegen lässt sich ein Entrainment der neuronalen Aktivität der zu desynchronisierenden Neuronenpopulation bezüglich der aufgezwungenen Frequenz erzielen.

Bei allen drei, oben beispielhaft beschriebenen, Kontrollmethoden bedingt eine in Abschnitt 5 beschriebene selbstregulierende Bedarfssteuerung eine Minimierung de Energieeintrags in die Zielpopulation. Dabei kann man vorzugsweise die einzig wichtigen Stimulationsparameter, die Stimulationsperiode T und damit die Zeitverzögerungen, zwischen den Einzelreizen, durch Messung der Frequenz der Nervenzellpopulation im Zielgebiet oder einer anderen, damit eng verbundenen Nervenzellpopulation anpassen.

Das Fehlen zeitaufwendiger Kalibrierung und die Stabilität der Wirkung auch bei stärkeren Frequenzschwankungen - insbesondere bei Methode 1 (permanente Stimulation) - hat wichtige Konsequenzen:

1. Schon intraoperativ lässt sich beim Einführen der Tiefenelektrode der Stimulationserfolg sofort überprüfen. Hierdurch kann das Auffinden des geeigneten Zielpunkts deutlich verbessert werden. Für die bisherigen bedarfsgesteuerten Verfahren benötigt man eine Kalibrierung, welche pro Elektrode länger als 30 Minuten dauert. Das ist intraoperativ nicht durchführbar und dem (nicht narkotisierten) Patienten nicht zumutbar.

2. Die neuen Stimulationsmethoden lassen sich auch bei neurologischen bzw. psychiatrischen Erkrankungen anwenden, bei denen pathologische Rhythmen stark schwankende Frequenzen aufweisen. Insbesondere lassen sich mit den neuen Methoden auch intermittent (d. h. kurzzeitig auftretende) Rhythmen desynchronisieren. Hieraus ergibt sich, dass die neuen Stimulationsmethoden bei weit mehr Erkrankungen zur Anwendung kommen können, vor allem auch bei den Epilepsien.

Mit der erfindungsgemäßen Vorrichtung können mit dem neuen Stimulationsverfahren folgende Krankheiten bzw. Symptome durch Desynchronisation geeigneter Hirnareale behandelt werden.

Bei allen neurologischen und psychiatrischen Erkrankungen, bei denen pathologische neuronale Synchronisation eine für die Ausprägung der krankheitsspezifischen Symptome eine relevante Rolle spielt, zum Beispiel: Morbus Parkinson, essentieller Tremor, Dystonie, Zwangserkrankungen, Tremor bei Multipler Sklerose, Tremor in Folge eines Schlaganfalls oder einer anderen, zum Beispiel tumorösen Gewebsschädigung, zum Beispiel im Bereich des Thalamus und/oder der Basalganglien, Choreoathetose und Epilepsie, wobei die Aufzählung nicht einschränkend sein soll.

Bei der zur Zeit verwendeten Standardmethode, der Hochfrequenz-Dauerstimulation, werden folgende Zielareale beispielhaft verwendet:

Bei Morbus Parkinson der Nucleus subthalamicus oder bei tremordominantem Morbus Parkinson der Thalamus, zum Beispiel der Nucleus ventralis intermedius thalami.
Bei essentiellem Tremor der Thalamus, zum Beispiel der Nucleus ventralis intermedius thalami.
Bei Dystonie und Choreoathetose der Globus pallidum internum bei Epilepsie der Nucleus subthalamicus, das Kleinhirn, thalamische Kerngebiete, zum Beispiel der Nucleus ventralis intermedius thalami, oder der Nucleus caudatus.
Bei Zwangserkrankungen die Capsula interna oder der Nucleus accumbens.

Bei der erfindungsgemäßen Vorrichtung können beispielsweise die für die jeweiligen Erkrankungen oben aufgeführten Zielareale gewählt werden. Weil bei der erfindungsgemäßen Vorrichtung entweder keine Kalibrierung notwendig ist oder die Kalibrierung sehr schnell durchgeführt werden kann, ergibt sich die Möglichkeit, im Rahmen der Elektrodenimplantation alternative Zielareale auszutesten, bei denen sich die desynchronisierende Wirkung der erfindungsgemäßen Vorrichtung noch besser entfalten lässt.

Die Erfindung umfasst ebenfalls eine Steuerung, welche die angegebene Funktionsweise der erfindungsgemäßen Vorrichtung steuert sowie die Verwendung der Vorrichtung und der Steuerung für die Behandlung der Krankheiten Morbus Parkinson, essentieller Tremor, Dystonie, Zwangserkrankungen, Choreoathetose, Tremor bei Multipler Sklerose, Tremor in Folge eines Schlaganfalls oder einer anderen, zum Beispiel tumorösen Gewebeschädigung, zum Beispiel im Bereich des Thalamus und/oder der Basalganglien, und Epilepsie.

Die erfindungsgemäße Vorrichtung kann sowohl als Implantat zur dauerhaften Therapie der obengenannten neurologischen und psychiatrischen Erkrankungen als auch für die intraoperative Zielpunkt-Diagnostik, das heißt, die intraoperative Auffindung des optimalen Zielpunkts für die Elektrodenimplantation, verwendet werden.

## Patentansprüche

**1.** Vorrichtung zur Desynchronisation von neuronaler Hirnaktivität, mit

- mindestens einem Sensor (3) zur Messung eines Signals, welches die zeitliche Entwicklung der Aktivität der zu desynchronisierenden Neuronenpopulation wiedergibt, sowie
- mindestens zwei Elektroden (2),

**gekennzeichnet durch**

- eine Steuerung (4), welche derart ausgestaltet ist, dass sie das Messsignal des Sensors (3) aufnehmen kann und jede der mindestens zwei Elektroden (2) mit dem Messsignal als Stimulationssignal oder mit einem bearbeiteten Messsignal als Stimulationssignal speisen kann.

**2.** Vorrichtung nach Anspruch 1,
**dadurch gekennzeichnet,**

- **dass** die Steuerung (4) Stimulationssignale erzeugen kann, die gegenüber den Messsignalen zeitverzögert sind.

**3.** Vorrichtung nach Anspruch 2,
**dadurch gekennzeichnet,**

- **dass** die Steuerung (4) Stimulationssignale an N Elektroden (2) mit wenigstens teilweise unterschiedlichen Zeitverzögerungen abgeben kann.

**4.** Vorrichtung nach Anspruch 2 oder 3,
**dadurch gekennzeichnet,**

- **dass** die Steuerung (4) Stimulationssignale erzeugen kann, deren Zeitverzögerungen im wesentlichen äquidistant sind.

**5.** Vorrichtung nach einem der Ansprüche 2 bis 4,
**dadurch gekennzeichnet,**

- **dass** die Steuerung (4) Stimulationssignale erzeugen kann, deren Zeitverzögerung einem Bruchteil oder einem Vielfachen der Bruchteile der Periode T der Messsignale entspricht.

**6.** Vorrichtung nach einem der Ansprüche 1 bis 5,
**dadurch gekennzeichnet,**

- **dass** die Steuerung (4) die Messsignale bearbeiten kann, wobei die Steuerung (4) die Messsignal filtern kann.

**7.** Vorrichtung nach einem der Ansprüche 1 bis 6,
**dadurch gekennzeichnet,**

- **dass** die Steuerung (4) die Messsignale bearbeiten kann, wobei die Steuerung (4) für die Messsignale eine Frequenzanalyse und/oder eine Wavelet-Analyse und/oder eine Bandpassfilterung und/oder Filterung und/oder eine Hilbert-Transformation in zeitlicher Domäne durchführen kann.

**8.** Vorrichtung nach einem der Ansprüche 1 bis 7,
**dadurch gekennzeichnet,**

- **dass** die Steuerung (4) die Messsignale bearbeiten kann, wobei die Steuerung (4) die Messsignale linear und/oder nichtlinear transformieren und/oder kombinieren kann.

**9.** Vorrichtung nach einem der Ansprüche 1 bis 8,
**dadurch gekennzeichnet,**

- **dass** die Steuerung (4) die Messsignale bearbeiten kann, wobei die Steuerung (4) die Messsignale verstärken kann.

**10.** Vorrichtung nach einem der Ansprüche 1 bis 9,
**dadurch gekennzeichnet,**

- **dass** die Steuerung (4) die Messsignale bearbeiten kann, wobei die Steuerung (4) die Polarität der Messsignale verändern kann.

**11.** Vorrichtung nach einem der Ansprüche 1 bis 10,
**dadurch gekennzeichnet,**

- **dass** die Steuerung (4) die Messsignale bearbeiten kann, wobei die Steuerung (4) die Messsignale zeitlich kodieren kann, wobei die Steuerung (4) insbesondere die Messsignale als Pulszüge, insbesondere als Hochfrequenzpulszüge, kodieren kann.

**12.** Vorrichtung nach einem der Ansprüche 1 bis 11,
**dadurch gekennzeichnet,**

- **dass** die Steuerung (4) aus den Messsignalen Einzelreize erzeugen kann.

**13.** Vorrichtung nach einem der Ansprüche 1 bis 12,
**dadurch gekennzeichnet,**

- **dass** die Steuerung (4) mindestens zwei Elektroden (2) mit unterschiedlichen Einzelreizen ansteuern kann.

**14.** Vorrichtung nach einem der Ansprüche 1 bis 13,
**dadurch gekennzeichnet,**

- **dass** die Steuerung (4) Unterschiede in der Leitungszeit zwischen dem Reizort einer einzelnen Elektrode (2) und dem Ort der von ihr stimulierten Neuronenpopulation detektieren kann.

**15.** Vorrichtung nach Anspruch 14,
**dadurch gekennzeichnet,**

- **dass** die Steuerung (4) bei der Berechnung der Zeitverzögerungen der Einzelreize der einzelnen Elektroden (2) und/oder bei der Bearbeitung der Messsignale die zugehörigen Leitungszeiten mit verrechnen kann.

**16.** Vorrichtung nach einem der Ansprüche 1 bis 15,
**dadurch gekennzeichnet,**

- **dass** die Steuerung (4) Signale für Gesamtreize an die Elektroden (2) abgeben kann, welche sich aus Signalen für Einzelreize zusammensetzen, wobei die Steuerung (4) die Reihenfolge und/oder die Art und/oder die Intensität und/oder den Energieeintrag der Einzelreize in einem Gesamtreiz mit einem deterministischen und/oder stochastischen Algorithmus ermitteln und variieren kann.

**17.** Vorrichtung nach einem der Ansprüche 1 bis 16,
**dadurch gekennzeichnet,**

- **dass** die Steuerung (4) die Stimulationsperiode T an die momentane Periode der zu desynchronisierenden Neuronenpopulation anpassen kann.

**18.** Vorrichtung nach einem der Ansprüche 1 bis 16, **dadurch gekennzeichnet,**

- **dass** die Steuerung (4) die Stimulationsperiode T an die mittlere Frequenz der zu desynchronisierenden Neuronenpopulation anpassen kann.

**19.** Vorrichtung nach einem der Ansprüche 2 bis 18, **dadurch gekennzeichnet,**

- **dass** die Steuerung (4) die Zeitverzögerung der Stimulationssignale an die Stimulationsperiode T anpassen kann.

**20.** Steuerung, **dadurch gekennzeichnet,**

- **dass** sie so programmiert ist, dass sie die Schritte für die Durchführung der Arbeitsweise der Vorrichtung nach einem der Ansprüche 1 bis 19 steuern kann.

**Claims**

**1.** A device for desynchronising neuronal cerebral activity, comprising

- at least one sensor (3) for sensing a signal representing the temporal development of the activity of the neuron population to be desynchronised, as well as
- at least two electrodes (2)

**characterized by**

- a controller (4) configured so that it can receive the sensed signal of the sensor (3) and supply each of the at least two electrodes (2) with the sensed signal as a stimulation signal or with a processed sensed signal as the stimulation signal.

**2.** The device as set forth in claim 1, **characterized in that**

- the controller (4) can generate stimulation signals time-delayed in relation to the sensed signals.

**3.** The device as set forth in claim 2, **characterized in that**

- the controller (4) can output stimulation signals to N electrodes time-delayed differingly at least in part.

**4.** The device as set forth in claim 2 or 3, **characterized in that**

- the controller (4) can generate stimulation signals time-delayed substantially equidistant.

**5.** The device as set forth in any of the claims 2 to 4, **characterized in that**

- the controller (4) can generate stimulation signals time-delayed corresponding to a fraction or a multiple of fractions of the period T of the sensed signals.

**6.** The device as set forth in any of the claims 1 to 5, **characterized in that**

- the controller (4) can process the sensed signals by the controller (4) being able to filter the sensed signal.

**7.** The device as set forth in any of the claims 1 to 6, **characterized in that**

- the controller (4) can process the sensed signals by the controller (4) being able to implement for the sensed signals a frequency analysis and/or a wavelet analysis and/or a bandpass filtering and/or filtering and/or a Hilbert transformation in time domains.

**8.** The device as set forth in any of the claims 1 to 7, **characterized in that**

- the controller (4) can process the sensed signals by the controller (4) being able to transform and/or combine the sensed signals linearly and/or non-linearly.

**9.** The device as set forth in any of the claims 1 to 8, **characterized in that**

- the controller (4) can process the sensed signals by the controller (4) being able to amplify the sensed signals.

**10.** The device as set forth in any of the claims 1 to 9, **characterized in that**

- the controller (4) can process the sensed signals by the controller (4) being able to change the polarity of the sensed signals.

**11.** The device as set forth in any of the claims 1 to 10,

**characterized in that**

- the controller (4) can process the sensed signals by the controller (4) being able to time code the sensed signals, particularly by coding the sensed signals as pulse trains, especially as high-frequency pulse trains.

**12.** The device as set forth in any of the claims 1 to 11, **characterized in that**

- the controller (4) can generate discrete stimuli from the sensed signals.

**13.** The device as set forth in any of the claims 1 to 12, **characterized in that**

- the controller (4) can activate at least two electrodes (2) with differing discrete stimuli.

**14.** The device as set forth in any of the claims 1 to 13, **characterized in that**

- the controller (4) can detect differences in the conduction time between the stimulus location of an individual electrode (2) and the location of the neuron population stimulated thereby.

**15.** The device as set forth in claim 14, **characterized in that**

- in calculating the time delay of the discrete stimuli of the individual electrodes (2) and/or in processing the sensed signals the controller (4) can include calculation of the corresponding conduction times.

**16.** The device as set forth in any of the claims 1 to 15, **characterized in that**

- the controller (4) can output signals for whole stimuli composed of signals for discrete stimuli to the electrodes (2) by the controller (4) being able to detect and vary the sequence and/or the nature and/or the intensity and/or the energy input of the discrete stimuli in a whole stimulus with a deterministic and/or stochastic algorithm.

**17.** The device as set forth in any of the claims 1 to 16, **characterized in that**

- the controller (4) can match the stimulation period T to the momentary period of the neuron population to be desynchronized.

**18.** The device as set forth in any of the claims 1 to 16, **characterized in that**

- the controller (4) can match the stimulation period T to the mean frequency of the neuron population to be desynchronized.

**19.** The device as set forth in any of the claims 2 to 18, **characterized in that**

- the controller (4) can match the time delay of the stimulation signals to the stimulation period T.

**20.** A controller (4) **characterized in that**

- it is programmed able to control the steps for implementing the response of the device as set forth in any of the claims 1 to 19.

**Revendications**

**1.** Dispositif pour la désynchronisation de l'activité neuronale du cerveau, comprenant :

- au moins un capteur (3) pour la mesure d'un signal, lequel redonne l'évolution temporelle de l'activité de la population de neurones devant être désynchronisée, ainsi que
- au moins deux électrodes (2),

**caractérisé par**

- une commande (4) qui est réalisée de telle sorte qu'elle peut réceptionner le signal de mesure du capteur (3) et qu'elle peut alimenter chacune des au moins deux électrodes (2) avec le signal de mesure comme signal de stimulation ou avec un signal de mesure traité comme signal de stimulation.

**2.** Dispositif selon la revendication 1, **caractérisé en ce que**

- la commande (4) peut générer des signaux de stimulation qui sont retardés en temps par rapport aux signaux de mesure.

**3.** Dispositif selon la revendication 2, **caractérisé en ce que**

- la commande (4) peut fournir des signaux de stimulation à N électrodes (2) avec des retards de temps tout du moins différents en partie.

**4.** Dispositif selon l'une ou l'autre des revendications 2 et 3, **caractérisé en ce que**

- la commande (4) peut générer des signaux de stimulation dont les retards de temps sont sensiblement équidistants.

**5.** Dispositif selon l'une des revendications 2 à 4, **caractérisé en ce que**

- la commande (4) peut générer des signaux de stimulation dont le retard de temps correspond à une fraction ou à un multiple des fractions de la période T des signaux de mesure.

**6.** Dispositif selon l'une des revendications 1 à 5, **caractérisé en ce que**

- la commande (4) peut traiter les signaux de mesure, et dans lequel la commande (4) peut filtrer le signal de mesure.

**7.** Dispositif selon l'une des revendications 1 à 6, **caractérisé en ce que**

- la commande (4) peut traiter les signaux de mesure, et dans lequel la commande (4) peut exécuter une analyse de fréquence et/ou une analyse des ondelettes et/ou un filtrage passe-bande et/ou un filtrage et/ou une transformée de Hilbert dans un domaine temporel pour les signaux de mesure.

**8.** Dispositif selon l'une des revendications 1 à 7, **caractérisé en ce que**

- la commande (4) peut traiter les signaux de mesure, et dans lequel la commande (4) peut transformer et/ou combiner les signaux de mesure linéairement et/ou non linéairement.

**9.** Dispositif selon l'une des revendications 1 à 8, **caractérisé en ce que**

- la commande (4) peut traiter les signaux de mesure, et dans lequel la commande (4) peut amplifier les signaux de mesure.

**10.** Dispositif selon l'une des revendications 1 à 9, **caractérisé en ce que**

- la commande (4) peut traiter les signaux de mesure, et dans lequel la commande (4) peut modifier la polarité des signaux de mesure.

**11.** Dispositif selon l'une des revendications 1 à 10, **caractérisé en ce que**

- la commande (4) peut traiter les signaux de mesure, et dans lequel la commande (4) peut coder temporellement les signaux de mesure, et dans lequel la commande (4) peut coder en particulier les signaux de mesure comme trains d'impulsion, en particulier comme trains d'impulsion à haute fréquence.

**12.** Dispositif selon l'une des revendications 1 à 11, **caractérisé en ce que**

- la commande (4) peut générer des excitations isolées à partir des signaux de mesure.

**13.** Dispositif selon l'une des revendications 1 à 12, **caractérisé en ce que**

- la commande (4) peut commander au moins deux électrodes (2) avec des excitations isolées différentes.

**14.** Dispositif selon l'une des revendications 1 à 13, **caractérisé en ce que**

- la commande (4) peut détecter des différences dans le temps de conduction entre le lieu d'excitation d'une électrode isolée (2) et le lieu de la population de neurones stimulée par elle.

**15.** Dispositif selon la revendication 14, **caractérisé en ce que**

- la commande (4) peut tenir compte des temps de conduction correspondants lors du calcul des retards de temps des excitations isolées des électrodes isolées (2) et/ou des lors du traitement des signaux de mesure.

**16.** Dispositif selon l'une des revendications 1 à 15, **caractérisé en ce que**

- la commande (4) peut fournir des signaux aux électrodes (2) pour des excitations globales, lesquels se composent de signaux pour des excitations isolées, et dans lequel la commande (4) peut déterminer et varier l'ordre séquentiel et/ou la nature et/ou l'intensité et/ou l'introduction d'énergie des excitations isolées dans une excitation globale avec un algorithme de détermination et/ou stochastique.

**17.** Dispositif selon l'une des revendications 1 à 16, **caractérisé en ce que**

- la commande (4) peut adapter la période de simulation T à la période momentanée de la population de neurones devant être désynchronisée.

**18.** Dispositif selon l'une des revendications 1 à 16, **caractérisé en ce que**

- la commande (4) peut adapter la période de simulation T à la fréquence moyenne de la population de neurones devant être désynchronisée.

**19.** Dispositif selon l'une des revendications 2 à 18, **caractérisé en ce que**

- la commande (4) peut adapter le retard de temps des signaux de simulation à la période de simulation T.

**20.** Commande, **caractérisée en ce que**

- elle est programmée de telle sorte qu'elle peut commander les étapes pour l'exécution de la marche à suivre du dispositif selon l'une des revendications 1 à 19.

4
11
12
10
13
5
6
1
7
9
8
2
3

Fig.1

1
0.5
0
0
5
10
15
20
25
30
Zeit [sec]

Fig. 2 a

0
-0.1
-0.2
-0.3
0
5
10
15
20
25
30
Zeit [sec]

Fig. 2 b

0.04
0.02
0
-0.02
-0.04
0
5
10
15
20
25
30
Zeit [sec]

Fig 2 c

Stimulusbeginn
neuronale Aktivität
Elektrode 1
Elektrode 2
Elektrode 3
Elektrode 4

Fig. 3

Stimulusbeginn
neuronale Aktivität
Elektrode 1
Elektrode 2
Elektrode 3
Elektrode 4

Fig. 4

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 10211766 A1
- WO 2003077985 A
- DE 10318071033


### In der Beschreibung aufgeführte Nicht-Patentliteratur


- **P. Tass.** Desynchronizing double-pulse phase resetting and application to deep brain stimulation. *Biological Cybernetics,* 2001, vol. 85 (5), 343-354
- **Elble R.J. ; Koller W.C.** Tremor. John Hopkins University Press, 1990